# EUROPEAN PATENT APPLICATION

(11) **EP 1 369 492 A1**
(43) Date of publication of application: **10.12.2003**
(21) Application number: 02027284.5
(22) Date of filing: 06.12.2002
(51) Int. Cl.: C12Q 1/68, C12N 9/02, C12R 1/44

(54) **Detection of staphylococcus spp**

(30) Priority: 07.06.2002 US 164758
(71) Applicant: DR. Chip Biotechnology Incorporation, Junan Township, Miaoli County, Taiwan 350 (TW)
(72) Inventor: Liu, Lu-Yieng, Science-Based Ind. Park, Hsinchu 300 (TW); Lee, Kan-Hung, Science-Based Ind. Park, Hsinchu 300 (TW); Terng, Harn-Jing, Science-Based Ind. Park, Hsinchu 300 (TW)
(74) Representative: Becker Kurig Straus

(57) **Abstract**

A novel nucleic acid containing an oligonucleotide selected from a member of the group consisting of SEQ ID NOs:1-25 and sequences complementary to SEQ ID NOs:1-25. The nucleic acid is 10-1000 nucleotides in length. Also disclosed is a method of detecting *Staphylococcus* spp.

## Description

The present invention relates to specific nucleic acid sequences selected from the *Staphylococcus* spp. *gap* gene region for detecting *Staphylococcus* spp. In particular, the present invention pertains to a method for detecting the presence or absence of *Staphylococcus* spp. In a biological sample.

Bacteria, in particular *Staphylococci,* are the causative agents of many opportunistic human and animal infections. For this reason, accurate and rapid identification of the presence of such bacteria in a sample is conducive to diagnosing, preventing and/or treating such infections.

Traditionally, the detection of a microorganism requires time-consuming growth of the microorganism in a culture medium, followed by its isolation and identification by conventional means. The entire process usually takes about 24-48 hours. So far, a variety of different methods for the detection of microorganisms have been developed, including miniaturized biochemical analyses, antibody- and DNA-based tests, and modified conventional assays. Yet, these methods suffer, in that they are still not fully reliable and in most cases too time consuming.

Therefore, a problem of the present invention resides in providing novel means for rapidly and easily detecting the presence of bacteria detrimental to the health of humans and animal in a sample.

This problem has been solved by providing a novel nucleic acid containing an oligonucleotide selected from a member of the group consisting of SEQ ID NOs:1-25 and sequences complementary to SEQ ID NOs:1-25, wherein the nucleic acid is 10-1000, e.g., 10-500, 10-300, 10-200, 10-100, 10-50, and 10-20 nucleotides in length. The nucleic acid can simply be a member of the group consisting of SEQ ID NOs:1-25 and/or sequences complementary to SEQ ID NOs:1-25. These nucleic acids can be used as sequencing primers, PCR primers and hybridization probes.

According to another embodiment, this invention features a pair of amplification primers: one primer contains an oligonucleotide selected from a member of the group consisting of SEQ ID NOs:28-52, and the other primer contains an oligonucleotide selected from a sequence complementary to the member. Each primer is 14-40, e.g. 14-30 or 14-20 nucleotides in length. These primers can be used to amplify a DNA template prepared from *Staphylococcus* spp.

Also within the scope of this invention is a method of detecting a target *Staphylococcus* species. The method involves (1) providing a sample having a nucleic acid from an unknown microorganism; (2) amplifying the nucleic acid with a pair of primers: one primer contains an oligonucleotide selected from a member of the group consisting of SEQ ID NOs:28-52, and the other primer contains an oligonucleotide selected from a sequence complementary to the member. Each primer is 14-40, e.g. 14-30 or 14-20 nucleotides in length; and (3) detecting an amplification product. Detection of the amplification product indicates the presence of the target *Staphylococcus* species. In one embodiment, the detecting step includes hybridizing the amplification product to a nucleic acid probe that is 10-1000 nucleotides in length and contains a sequence selected from a member of the group consisting of SEQ ID NOs:1-27 and sequences complementary to SEQ ID NOs:1-27. The nucleic acid probe can simply be a member of the group consisting of SEQ ID NOs:1-27 and sequences complementary to SEQ ID NOs:1-27.

Further within the scope of this invention is a kit for detecting *Staphylococcus* spp.. The kit contains one or more of the nucleic acids described above. It can also include other components such as a DNA polymerase, a PCR buffer, or a solid support on which one or more of the above-described probes are immobilized.

The present invention provides a fast, accurate, and sensitive method for *Staphylococcus* spp. detection and typing. The details of one or more embodiments of the invention are set forth in the accompanying description below. Other advantages, features, and objects of the invention will be apparent from the detailed description, and from the claims.

The present invention is based on the findings that the *Staphylococcus* spp. *gap* gene region can be used for identification of specific *Staphylococcus* species. The *gap* gene regions of 27 *Staphylococcus* spp. are amplified using a primer pair (GF-1: 5'-atggttttggtagaattggtcgttta-3' and GR-2: 5'-gacatttcgttatcataccaagctg-3'; Yugueros *et al*., 2000, *J. Clinical Microbiology* 38: 4351-4355) located in the conserved region of the *gap* gene. Sequences of the amplified DNA fragments are shown below:
(1) *Staphylococcus arlettae* 
1   atggttttggtagaattggtcgtttagcatttagaagaattcaaaacgttgatggaatcg 60
61  acgttgtagcagtaaacgacttaacagatgatgaaatgttagcacacttattaaaatatg 120
121 acactatgcaaggacgtttcacaggagaagttgaagtagaaaatgacggtttccgtgtaa 180
181 atggtcaagaagttaaatcattctcagaaccagatccaaataaattaccatggggcgact 240
241 tagatatcgatgttgttttagaacgtactggtttattcgcagacaaagataaagcatcag 300
301 ctcatatcgacgcaggtgcgaaaaaagtattaatctctgctccagcatcaggtgatttaa 360
361 aaacaatcgtattcaacactaaccacaatgaattagatggttctgaaacagttgtatcag 420
421 gtgcttcatgtactactaactcattagctccagttgctaaagtattaaacgatgacttcg 480
481 gtttagtagaaggtttaatgactactatccatgcttacactggtgaccaaagcacacaag 540
541 acgctcctcacagaaaaggtgacaaacgtcgtgcgcgtgcagctgctgaaaacattattc 600
601 ctaactcaacaggtgctgctaaagcaatcggtttagttattcctgaaatcgatggtaaat 660
661 tagatggtggcgctcaacgtgtaccagtagctactggttcattaactgaattaacagttg 720
721 tattagacaaagaagtaactgttgaagacgttaatagtgcaatgaaaaatgcttcaaacg 780
781 aatcatttggttacactgaagacgaaatcgtttcttctgacgtaatcggtatgacttacg 840
841 gttcattattcgatgctacacaaactcgtgtaatgactgttggtaaccaacaaatggtta 900
901 aagtagcagcttggtatgataacgaaatgtc (SEQ ID NO:1)
(2) *Staphylococcus auricularia* 
1 atggttttggtagaattggtcgtttagcattcagaagaattcaaaatgttgaaggcattg 60
61 acgtagtagcagttaatgacttaacagatgacgatatgttagcacacttattaaaatacg 120
121 acacaatgcaaggtcgtttcacaagtgaagttgaaatcgtagaaggcggtttccgcgtta 180
181 acggtcaagaagtgaaatcattcgacgaaccagatccaagcaaattaccttggaaagact 240
241 tagacatcgacgttgtacttgaatgtactggtttattcacagacaaagaaaaagcagaag 300
301 cacacatcgacgcaggtgctaaaaaagtattaatctctgcaccagctaaaggtgacgtta 360
361 aaacagttgtttataacactaaccacgatacacttgatggtacagaaacagttgtttcag 420
421 gtgcttcatgtacaacaaactcattagcaccagttgctaaagtacttaacgatgaattcg 480
481 gtttagtagaaggattcatgactacaatccacgcatacactggtgaccaaaatacacaag 540
541 atgcaccacacaaaaaaggtgacaaacgccgtgcacgtgcagctgctcaaaacatcatcc 600
 
601 ctaactctacaggtgctgcaaaagcaatcggtaaagttatcccagaaatcgacggtaaat 660
661 tagacggcggtgcacaacgtgtccctgttgctactggttcattaacagaattaacagtag 720
721 tattagaaaaagatgtttcaatcgaagacgtaaacaacgcaatgaaaaatgcatcaactg 780
781 aatcattcggttacacagatgacgaaatcgtttcttctgacgtcatcggtatgacttatg 840
841 gttcattattcgacgcaactcaaactcgtgtcatgactgttggcgatcaccaattaatca 900
901 aagttgcagcttggtatgataacgaaatgtc (SEQ ID NO:2)
(3) *Staphylococcus caprae* 
1 atggttttggtagaattggtcgtttagcattcagaagaatccaagatgtagaaggtctta 60
61 aagtagtagcagttaacgacttaacagacgatgaaatgttagctcatttattaaaatatg 120
121 acactatgcaaggacgcttcactggagaagttgaagttatcgaaggcggattccgcgtaa 180
181 acggtaaagaaattaaatcattcgacgaaccagatgcaaacaaattaccttggggtgacc 240
241 ttgacatcgatgtagtattagaatgtactggtttctacactgataaagataaagcacaag 300
301 cacatatcgatgcaggtgctaaaaaagtattaatctcagctccagctactggtgatttaa 360
361 aaacaatcgtattcaatactaaccatgacgaattagatggttcagaaacagttgtatcag 420
421 gtgcttcttgtacaactaactcattagctcctgttgctaaagtattaaatgatgaattcg 480
481 gtttagttgaaggtttaatgacaactatccacgcttacacaggtgaccaaaatactcaag 540
541 acgcacctcacagaaaaggtgacaaacgtcgtgctcgtgcagcagcagaaaacattatcc 600
601 ctaactcaacaggtgctgctaaagcaatcggtaaagtaattcctgaaatcgacggtaaat 660
661 tagacggtggtgcacaacgtgttccagttgctacaggttcattaacagaattaactgtag 720
721 tattagaaaaacaagatgtaactgctgatcaagttaacgaagcaatgaaacaagcttctg 780
781 acgaatcattcggttacactgaagatgaaatcgtatcttctgacgttgtaggtatcactt 840
841 atggttcattattcgatgcaactcaaactcgtgtaatgactgttggtgaccgtcaattag 900
901 ttaaagtagcagcttggtatgataacgaaatgtc (SEQ ID NO:3)
(4) *Staphylococcus capitis* 
1 atggttttggtagaattggtcgtttagcattcagaagaatccaagatgtagaaggtctag 60
61 aagtagttgcagttaacgacttaacagatgatgaaatgttagctcacttattaaaatatg 120
121 acactatgcaaggtcgcttcactggagaagttgaagtcatcgatggtggattccgtgtta 180
181 acggtaaagaaattaaatcattcgatgaaccagatgcaagcaaattaccttggggagacc 240
241 tagacatcgacgtagtattagaatgtactggtttctacactgacaaagataaagcacaag 300
301 ctcacatcgatgcaggtgctaaaaaagtattaatctctgctccagctacaggtgatttaa 360
361 aaacaatcgtattcaacactaaccatgatgaattagatggttctgaaacagttgtatcag 420
421 gtgcttcttgtacaactaactcattagcaccagttgctaaagttttaaatgatgagtttg 480
481 gtttagttgaaggtttaatgactactatccacgcttatactggtgaccaaaatactcaag 540
541 atgctcctcacagaaaaggcgacaaacgtcgtgctcgtgcagcagcagaaaacattatcc 600
601 ctaactctacaggtgctgctaaagcaattggtaaagttattccagaaatcgatggtaaat 660
661 tagacggtggagctcaacgtgttccagttgcaacaggttcattaactgaattaacagtag 720
721 tattagataaacaagatgtaacagctgatcaagtaaatgaagcaatgaaacaagcttctg 780
781 acgaatcattcggttacactgaagatgaaatcgtaccttctgacgttgtaggtatgactt 840
841 atggttcattattcgacgctactcaaactcgtgtaatgactgttggtgaccgtcaattag 900
901 ttaaagttgcagcttggtatgataacgaaatgtc (SEQ ID NO:4)
(5) *Staphylococcus carnosus* 
1 atggttttggtagaattggtcgtttagcatttagaagaattcaagatgttgaaggtatcg 60
61 atgtagtagcagtaaacgacttaacagatgatgaaatgttagcacacttattaaaatacg 120
121 atacaatgcaaggacgtttcactgaagaagttgaagttgtagatggcggattccgcgtga 180
181 atggtaaagaagttaaatcattcgaagaaccagatgcaagcaaattaccttggaaagatt 240
241 tagacattgatgttgtattagaatgtacaggtttctatacaagtgaagaaaaagcaaaag 300
301 ctcatatcgatgcaggcgctaaaaaagtattaatttcagcaccagctactggtgatgtta 360
361 aaacaatcgtttataacgtaaaccaagatactttagacagctctgacgtaatcgtttcag 420
421 gtgcttcttgtactacaaactctcttgctcctgtagcacaagttttaaatgacagctttg 480
481 gtttagttgaaggtttcatgactactatccatgcttatactggtgaccaaaatactcaag 540
541 atggtccacacagaaaaggtgacaaacgtcgtgctcgtgcagcagctgaaaacatcgttc 600
601 caaactcaactggtgcagctaaagcaattggtaaagtaattcctgaaatcgacggaaaat 660
661 tagacagcggcgctcaacgtgttcctgttgcaactggttcattagctgaattaacagttg 720
721 tattagataaagacgttacagttgacgaagtaaacgaagcaatgaaacaagcttctaacg 780
781 aatcattcggttacaacgaagacgaaatcgtatcttcagatgttgtaggtatgacattcg 840
841 gttcattatttgatgcaactcaaactcgtgtaatgactgtttcaggccgtcaattagtta 900
901 aagtagcagcttggtatgataacgaaatgtc (SEQ ID NO:5)
(6) *Staphylococcus chromogenes* 
1 atggttttggtagaattggtcgtttagcattcagaagaattcaagacgtagaaatattg 60
61 aggttgtagctgtaaacgatttaacagacgacgatatgcttgcacatttattgaaatatg 120
121 acacaatgcaaggtcgttttactgaagaagtagatgtaattgatggtggtttccgcgtaa 180
181 atggtaaagaagtgaaatcattctctgaaccagaaccatcaaaattaccatggaaagatc 240
241 ttgacgtagatgttgttttagaatgtacaggtttctttacatcaaaagaaaaagcagaag 300
301 ctcacattgaagcaggtgctaaaaaagtattaatttctgcaccaggaactggcgatctta 360
361 aaacaatcgtatataatgtcaaccatgaagaattagacggttctgaaacagttgtatcag 420
421 gtgcttcttgtacaacaaactcattagcaccagtagcaaaaactttaaatgatgaatttg 480
481 gtatcgttgaaggtttaatgactacaattcacgcatacactggtgaccaaaatacacaag 540
541 actcaccacacagaaaaggtgacaaacgtcgtgcacgtgcagctgcagaaaacattattc 600
601 ctaactcaacaggtgctgcgaaagcaatcggtttagttatccctgaattgatggaaaat 660
661 tagacggtggcgcacaacgtgtaccagtagcaacaggttcattaactgaattaacagttg 720
721 ttttagataaagaagtatcagtagaagacgttaacaatgcaatgaaaaatgcaacaaacg 780
781 aatcattcggttacactgaagacgaaattgtatcttcagatgttgtaggcatgactttcg 840
841 gagcattatttgatgcaactcaaacacgtgtaatgactgttggcgaccgtcaattagtta 900
901 aagtagcagcttggtatgataacgaaatgtc (SEQ ID NO:6)
(7) *Staphylococcus cohni* 
1   atggttttggtagaattggtcgtttagcatttagaagaattcaagatgtagaaggaatcg 60
61  atgtagtagcagtaaacgacttaacagatgatgaaatgttagctcatttattaaaatatg 120
121 acactacacaaggtcgcttcacaggagaagttgaagttgaagaaatggtttccgcgtta 180
 
181 acggattagaagttaaatcatttctgagccagatccaagtaaattaccttggggagact 240
241 tagatatcgatgttgtattagaatgtactggtttattcacagataaagaaaaagcagaag 300
301 cacacatcaatgcaggcgctaaaaaagtattaatttcagcaccagctaaaggtgacttaa 360
361 aaacaatcgtatacaacactaaccacgacactttagatggttcagaagatgttgtttcag 420
421 gtgcttcatgtactactaactcattagcaccagttgctaaagttttaaatggtgaattcg 480
481 gcttaatcgaaggtttcatgactacaatccacgcatatactggtgaccaaagcacacaag 540
541 atgcgcctcacagaaaaggtgacaaacgtcgtgcgcgtgcagctgctgaaaacattatcc 600
601 ctaactcaacaggtgctgctaaagctatcggcctagtaattcctgaaattgatggtaaat 660
661 tagacggtggagcacaacgtgtaccagttgcaactggttcattaactgaattaacagtag 720
721 tattagataaaaatgtaagtatcgaagacgtaaataacgcaatgaaaaatgcatctaacg 780
781 aatcattcggttatactgaagacgaaatcgtttcttctgacgtaatcggaatgacatacg 840
841 gttcattatttgatgcaactcaaacacgtgttatgactgttggagatcgtcaattagtta 900
901 aagtagcagcttggtatgataacgaaatgtc (SEQ ID NO:7)
(8) *Staphylococcus delphini* 
1 atggttttggtagaattggtcgtttagcatttagaagaattcaagatgtggaaaatatcg 60
61 acgttgtagcagtaaacgatttaacagacgacgatatgcttgcacacttattaaaatatg 120
121 actcaacacaaggtcgttttactgaagaagtagaagtaattgacggtggtttccgtgtaa 180
181 atggtaaagaagtgaaatcattctctgaaccagaacctaaaaacttaccatggggcgagt 240
241 tagacatcgacgtggtattagaatgtactggtttcttcactgataaagaaaaagctgaag 300
301 cacacatcgaagcaggcgcgaaaaaagtattaatttctgcaccagctaaaggtgacctta 360
361 aaacagttgtatataacgttaaccacgaaattttagatggtactgaaacagttgtttctg 420
421 gtgcttcatgtacaacaaactcattagcacctgttgcaaaaactttacaagacaacttcg 480
481 gtatcgttgaaggtttaatgacaacaattcacgcttacactggtgaccaaaacacattag 540
541 acgcacctcacagaaaaggtgacaaacgtcgtgcgcgtgcagctgctgaaaacatcatcc 600
601 ctaactcaactggtgctgcgaaagcaatcggtttagttattcctgaaatcgatggtaaat 660
661 tagacggtggtgcacaacgtgttcctgtagcaacaggttcattaactgaattaactgtag 720
721 tattagaaaaagaagtctctgttgaagaagttaacaaagtcatgaaagaggcaactaacg 780
781 aatcattcggttacactgaagacgaaatcgtttcttcagacgttgttggtatgactttcg 840
841 gtgctttattcgacgcaactcaaacacgtgtaatgactgttggcgaccgtcaattagtta 900
901 aagttgcagcttggtatgataacgaaatgtc (SEQ ID NO:8)
(9) *Staphylococcus epidermidis* 
1   atggttttggtagaattggtcgtttagcatttagaagaattcaagacgtagaaggtttag 60
61  aagtggttgcagtaaacgacttaactgatgacgatatgttagcacacttattaaaatatg 120
121 acactatgcaaggtcgcttcactggtgaagtagaagttatcgacggtggattccgtgtta 180
181 acggtaaagaagttaaatcattcgatgaaccagatgcaagcaaattaccttggaaagact 240
241 tagatatcgacgtagtattagaatgtactggtttctatactgacaaagataaagcacaag 300
301 ctcatgttgac[?]caggtgctaaaaaagtattaatctcagctccagcaactggtgacttaa 360
 
361 aaacaatcgtttacaacactaaccacgatgaattagatggttctgaaacagtagtatcag 420
421 gtgcatcatgtactactaactcattagctcctgtagctaaagtgttaagtgatgagttcg 480
481 gtttagttgaaggtttaatgacaactatccacgcttacactggtgaccaaaatactcaag 540
541 atgctccacacagaagaggcgacaaacgtcgtgctcgtgctgcagcagaaaacatcatcc 600
601 ctaactcaactggtgctgctaaagcaatcggtaaagtaattcctgaaatcgacggaaaat 660
661 tagacggtggtgcacaacgtgttccagttgcaactggttcattaactgaattaacagttg 720
721 tattagaaaaagacgtaacagttgaacaagttaacgaagcaatgaaacaagcttctgacg 780
781 aatcattcggttacactgaagacgaaatcgtatcttctgatgtagttggtatgacttacg 840
841 gttcattattcgatgcaactcaaactcgtgtaatggctgttggtggtcgtcaattagtta 900
901 aagtagcagcttggtatgataacgaaatgtc (SEQ ID NO:9)
(10) *Staphylococcus equorum* 
1   atggttttggtagaattggtcgtttagcatttagaagaattcaagacgtagaaggtattg 60
61  acgtagtagcagttaacgatttaacagatgacgaaatgttagctcatttattaaaatatg 120
121 acactacacaaggtcgcttcacaggagaagttgaagtagaaaaagacggattccgtgtaa 180
181 atggacaagaagttaaatcattctcagaacctgaaccaagtaaattaccttggaaagatt 240
241 tagacatcgatgttgttttagaatgtactggtttcttcgctgataaagaaaaagcagaag 300
301 ctcacattgacgctggcgctaaaaaagtattaatctctgcaccagcaacaggcgacttaa 360
361 aaacaatcgtttataacactaaccacagtgaattagatggttcagaaacagttgtttcag 420
421 gtgcttcatgtactactaactcattagctccagtagctaaagttttaaatgacgacttcg 480
481 gcttagttgaaggtttcatgactactattcacgcatatactggtgaccaaagtactcaag 540
541 atgctccacacagaaaaggcgacaaacgccgtgcacgtgcagctgctgaaaacatcatcc 600
601 ctaactcaacaggtgctgctaaagcaattggtttagtaatccctgaaatcgatggtaaat 660
661 tagacggtggcgctcaacgtgttccagttgctactggttcattaactgaacttacagttg 720
721 tattagaaaaagacgtaagcgttgaagacgttaacgcagcaatgaaaaatgcttcagacg 780
781 aatcatttggttacactgaagacgaaatcgtttcttctgacgtaatcggtatgacttacg 840
841 gttcattattcgatgcaacgcaaactcgtgttatgacagttggagatcaccaattagtta 900
901 aaatagcagcttggtatgataacgaaatgtc (SEQ ID NO:10)
(11) *Staphylococcus gallinarum* 
1   atggttttggtagaattggtcgtttagcattcagaagaattcaaaacgttgaaggaatcg 60
61  acgtagtagcagtaaatgacttaacagatgacgaaatgttagctcacttattaaaatatg 120
121 atactatgcaaggtcgcttcactggagaagttgaagttgaaaaagacggtttccgtgtta 180
181 acggtcaagaagttaaatcattctctgagccagacccaagtaaattaccatggggtgact 240
241 tagacatcgatgtagtattagaatgtactggtttcttcgctgacaaaactaaagcagaag 300
301 ctcacatcaatgcaggtgctaaaaaagtattaatctcagctccagcaactggtgacttga 360
361 aaacaatcgttttcaacactaaccataacgaattagatggtacagaaacagttgtttcag 420
421 gtgcttcatgtactactaactcattagctccagtagctaaagtattaaatgatgactttg 480
481 gtttagttgaaggtcttatgactacaattcacgcttacactggtgaccaaaatacacaag 540
541 atgctccacatgctaaaggtgacaaacgccgtgctcgtgcagctgctgaaaatatcatcc 600
601 ctaactcaactggtgctgctaaagcaatcggtaaagttatccctgaaattgacggcaaat 660
661 tagacggtggtgcgcaacgtgtaccagttgctactggttctttaactgaattaacagttg 720
721 tattagaaaaagacgtaagcgttgaagacgttaacaatgcaatgaaaaacgcttcaaacg 780
781 aatcattcggttacactgaagacgaaatcgtatcttctgacgtagttggtatcacttacg 840
841 gtccattattcgatgcaacacaaactcgtgtaatgactgttggcgatcgtcaattagtta 900
901 aagttgcagcttggtatgataacgaaatgtc (SEQ ID NO:11)
(12) *Staphylococcus haemolyticus* 
1 atggttttggtagaattggtcgtttagcatttagaagaattcaagacgtaggaggtattg 60
61 aagtagttgcagtaaacgacttaacagacgacgaaatgttagctcatttattaaaatatg 120
121 acactatgcaaggtcgtttcacaggagaagttgaagttgttgatggtggtttccgcgtaa 180
181 atggtaaagaagttaaatcatacgaagaaccagatgcaagcaaattaccttggggcgatt 240
241 tagatatcgacgtagtattagaatgtactggtttctatacagataaagaaaaagcagaag 300
301 cacacatcaatgcaggtgctaaaaaagtattaatctctgcaccagctcaaggtgatgtaa 360
361 aaacaatcgtattcaacactaaccacaatgacttagatggttcagaaacagttgtttcag 420
421 gtgcatcatgtactactaactcattagcaccagttgctaaagtattaagtgatgaatttg 480
481 gtttagttgaaggtttaatgacaactattcacgcatacactggtgaccaaatgactcaag 540
541 acggtccacataaaaaaggtgacaaacgtcgtgcacgtgcagcagctcaaaacatcgtac 600
601 ctaactcaacaggtgctgcaaaagcaatcggtaaagttattcctgaaatcgatggtaaat 660
661 tagacggtggtgctcaacgtgtaccagttgctacaggttcattaactgaagtgacagttg 720
721 tattagaaaaagacgttactgttgaagacgttaacaaagcaatgaaaaacgcttcaaacg 780
781 aatcatttggttacactgaagacgaaatcgtttcttctgacgtagttggcatgacttacg 840
841 gttcattattcgatgctactcaaactcgtgtaatgtctgttggtgaccgtcaattagtta 900
901 aagttgcagcttggtatgataacgaaatgtc (SEQ ID NO:12)
(13) *Staphylococcus hominis* 
1   atggttttggtagaattggtcgtttagcattcagaagaattcaagacgtagaaggtattg 60
61  aagtagttgcagtaaacgacttaacagacgacgaaatgttagctcatttattaaaatatg 120
121 acaGtatgcaaggtcgctttaacggagacgtagaagtagttgaaggtggtttccgtgtaa 180
181 atggtaaagaagttaaatcttttgaagaaccagatgcaagtaaattaccatggggcgatt 240
241 tagatatcgacgtagtattagaatgtactggtttctatacagaaaaagaaaaagctgaag 3[?]0
 
301 cacacattaatgcaggagctaaaaagtattaatttctgctccagccaaaggtgatgtta 360
361 aaactatcgtatttaacacaaaccacaaagacttagatggatctgaaacagtagtatcag 420
421 gtgcttcatgtactacaaactcattagcaccagttgctaaagttttaaatgacgaatttg 480
481 gtattgttgaaggtttaatgacaactatccatgcttacactggtgaccaaatgactcaag 540
541 atggtccacacagaaaaggtgacaaacgtcgtgcacgtgcagcagcacaaaacatcgtac 600
601 ctaactcaacaggtgcagctaaagctatcggtaaagtaattcctgaaatcgatggtaaat 660
661 tagatggtggagcacaacgtgtaccagtagctactggttcattaactgaagtaacagttg 720
721 tattagaaaaagaagtaacagttgaagatgttaacaaagcaatgaaaaatgctgctgacg 780
781 aatcattcggttacactgaagatgaaatcgtatcatcagatgttgctggtatgaactttg 840
841 gttcattattcgatgcaactcaaactcgtgtcatctcagttggcgacaaacaattagtta 900
901 aagtagcagcttggtatgataacgaaatgtc (SEQ ID NO:13)
(14) *Staphylococcus hyricus* 
1 atggttttggtagaattggtcgtttagcatttagaagaattcaagacgtagaaacattg 60
61 aggtagtagctgtcaatgatttaactgacgacgacatgcttgcacatttattaaaatatg 120
121 acacgatgcaaggacgttttactgaagaagtagatgtaattgatggtggtttccgcgtaa 180
181 atggtaaagaagtgaaatcattctctgaaccagaaccatctaaattaccttggaaagact 240
241 tagaagtagatgttgttttagaatgtactggtttcttcacatctaaagaaaaagctgaag 300
301 cacacattgaagcaggcgctaaaaaagtcttaatttcagcaccaggtactggtgatctta 360
361 aaacaattgtatataacgttaaccatgaagaattagacggttcagaaacagttgtttcag 420
421 gtgcgtcttgtactacaaactcattagctccggtagcgaaaacattacacgatgaatttg 480
481 gtattgttgaaggtttaatgactacaattcacgcttatacaggtgaccaaaatacgcaag 540
541 actcacctcacagaaaaggtgacaaacgtcgtgcacgtgcagctgctgaaaacatcatcc 600
601 ctaactcaacaggtgctgcaaaagcaatcggtttagttattccagaaattgctggtaaat 660
661 tagatggtggcgcgcaacgtgtaccagttgctacaggttcattaacagaattaactgttg 720
721 ttttagaaaaagaagtatctgttgaagaagttaacaatgcaatgaaaaatgcaactaatg 780
781 aatcattcggttacactgaagatgaaatcgtctcttctgacgttgtaggtatgacgtttg 840
841 gtgcattattcgacgcaactcaaacacgcgttatgactgttggcgatcgtcaattagtta 900
901 aagttgcagcttggtatgataacgaaatgtc (SEQ ID NO:14)
(15) *Staphylococcus intermedius* 
1   atggttttggtagaattggtcgtttagcattccgtcgtattcaaaatgtggaaggaattg 60
61  aagttgttgcaatcaatgacttaacagacgctaaaatgttagctcatctgttaaaatatg 120
121 atacaactcaaggccgttttgacggtgaagtagaagtacatgatggtttcttcaaagtaa 180
181 acggtaaagaagttaaagtattagctaaccgtaacccagaagaacttccatggggtgaac 240
241 taggagtagacatcgttcttgaatgtactggtttcttcacagcacaagacaaagctgaat 300
301 tacacattaaagctggcgctaaaaaagttgttatctccgctccagcaactggcgacatga 360
361 aaacaatcgtttacaatgtaaaccatgaaacattagacggaactgaaacagttatttctg 420
421 gtgcaagctgtactactaactgtttagctccaatggctaaagttttagaagacaaatttg 480
481 gtgttgttgaaggcctaatgactacaattcacgcatacactggtgaccaaaatacattag 540
541 acgctccacatccaaaaggtgacttccgtcgtgctcgtgctgctgcagaaaatatcatcc 600
601 ctaatacaactggtgctgcaaaagctatcggtgaagtattaccaagccttaaaggtaaat 660
 
661 tagacggagcagctcaacgtgttccagttccaactggttcccttactgaattagtaacag 720
721 ttcttaacaaaaaagttactgttgatgaagtaaatgcagctatggaagcagcttctgatc 780
781 cagaaacattcggttacactaatgacgcaatcgtttcttctgatatcaaaggtatgactt 840
841 tcggttctttatttgacgaaactcaaacaaaagttcttacagttggcgatcaacaattag 900
901 ttaaaactgcagcttggtatgataacgaaatgtc (SEQ ID NO:15)
(16) *Staphylococcus kloosii* 
1 atggttttggtagaattggtcgtttagcatttagaagaattcaaaacgttgacggaatcg 60
61 atgtagtagcagttaacgacttaacagatgacgaaatgttagcacacttattaaaatatg 120
121 acacaatgcaaggtcgtttcactggagaagttgaagttgaagaaaacggcttccgcgtaa 180
181 atggtcaagaagttaaatcattctctgaaccagatccaagtaaattaccatggggcgact 240
241 tagatatcgatgttgtcttagaatgtactggtttatttgctgataaagataaagcttcag 300
301 ctcatatcgatgcaggcgctaaaaaagttttaatttcagctccagctacaggcgacttaa 360
361 aaacaatcgtttacaacactaaccacaacgaattagacggttcagaatcagtagtatcag 420
421 gtgcttcatgtactactaactcattagctccagtagctaaagttttaaatgatgaattta 480
481 gtttagttgaaggtttaatgacaactatccacgcttacactggtgaccaaagcacacaag 540
541 atgctcctcacagaaaaggtgacaaacgtcgtgctcgtgcagcagcagaaaacatcatcc 600
601 ctaactcaacaggtgctgcaaaagcaatcggtttagttattcctgaaatcgacggaaaat 660
661 tagacggtggcgcacaacgtgttccagttgcaacaggttcattaactgaattaacagttg 720
721 tattagaaaaagacgtaagtgttgaagatgtaaacaacgcaatgaaaaatgcttcaaacg 780
781 aatcatttggttacactgaagacgaaatcgtttcttctgacgtaatcggtatgacttacg 840
841 gttcattattcgacgctacacaaactcgtgtaatgactgttggtgactcgtcaattagtt 900
901 aaagttgcagcttggtatgataacgaaatgtc (SEQ ID NO:16)
(17) *Staphylococcus lentus* 
1 atggttttggtagaattggtcgtttagcatttagaagattacaagaagtagaaaatatcg 60
61 aagtagtagcaatcaacgatttagcagatgacgctatgttagctcatttattaaaatacg 120
121 attctacacaaggtcgtttcaaagatgaagtagaagtaattgaaggcggattccgtgtaa 180
181 acggtaaagaaatcaaaactttcgaaaatcctaaccctaaagaattaccctggggagact 240
241 tagacatcgatgtagtattagaatgtactggtttcttcgctgataaagaaaaagctcatg 300
301 ctcacatcgccgcaggtgctaaaaaagtattaatttcagctccagcttcaggcgacttga 360
361 aaacaatcgtatacaatgttaaccatgatgaattagacggttcagaagaaatcgtatctg 420
421 gtgcatcttgtactactaactgtttagctccaatggctaaagtattaaatgatgaattcg 480
481 gtatcgttgaaggattaatgatgacaattcatgcttatactggtgaccaaaatacactag 540
541 atgctccacatgctaaaggtggcttccgtcgtgctcgtgcagcagctgaaaacatcgtac 600
601 ctaactcaactggtgcagctaaagcaattggcttagttatcccagaattaaaggtaaat 660
661 tagatggatcagctcaacgtgttccagtagcaactggttcagtaactgaattaacagcag 720
721 tattagataaagaagtatctatagaagaaatcaacgaagcaatgaaaaatgctacaaatg 780
781 attcattcggatacactgaagacgaaatcgtttcttctgatgttattggcatcacttacg 840
841 gttcattattcgacgcaactcaaactcgtgtaatgacagttggagaccgtcaattagtta 900
901 aaactgcagcttggtatgataacgaaatgtc (SEQ ID NO:17)
(18) *Staphylococcus lugdunesis* 
1   atggttttggtagaattggtcgtttagcatttagaagaattcaagacgtagaaggcatcg 60
61  aggtagtagcagtaaacgacttaacagatgatgatatgttagcgcatttattaaaatatg 120
121 acactatgcaaggtcgcttcactagcgaagttgaagttgttgatggtggtttccgtgtaa 180
181 atggtaaagaagttaaatcatttgaagaaccagacgcaagcaaattaccatggggtgacc 240
241 taggtgttgacgtggtattagaatgtactggattctatacagataaagaaaaagctgaag 300
301 cacacattcatgcaggtgctaaaaaagtattaatttctgcgccagctaaaggtgacgtta 360
361 aaactatcgtttacaacactaaccatagtgacttagacggttcagaaacagttgtatcag 420
421 gtgcttcatgtacaactaactctttagcaccagtagctaaagtaatcagcgatgaatttg 480
481 gtttagtagaaggtttaatgacaactattcatgcatacactggtgaccaaatgactcaag 540
541 atggtccacacagaaaaggtgacaaacgtcgtgctcgtgcagctgcacaaaacatcgtac 600
601 ctaactcaactggtgctgctaaagcaatcggtaaagttattcctgaaatcgatggtaaat 660
661 tagacggtggtgcgcaacgtgtacctgtagctacaggttcattaactgaattaactgtag 720
721 tattagaaaaacaagatgtaacagtagaacaagtaaacgaagcgatgaaaaagcttcta 780
781 acgaatcattcggttacaatgaagatgaaattgtttcttctgacgtagttggtatgactt 840
841 acggttcattatttgatgcaacacaaactcgtgtaatgtcagttggtggccgtcaattag 900
901 ttaaagttgcagcttggtatgataacgaaatgtc (SEQ ID NO:18)
(19) *Staphylococcus piscifermentans* 
1 atggttttggtagaattggtcgtttagcattcagaagaattcaagatgttgaaggtatcg 60
61 atgtagtagcagtaaacgacttaacagatgatgaaatgttagcgcatttattaaaatacg 120
121 atacaatgcaaggacgtttcacagaagaagttgaagttgtagatggcggattccgtgtga 180
181 atggtaaagaagttaaatcattcgaagaaccagatgcaagcaaattaccttggaaagatt 240
241 tagacattgatgttgtattagaatgtactggtttctatacaagtgatgaaaaagctaaag 300
301 cacatatcgacgcaggtgctaaaaaagtattaatttctgctccagcaactggcgatgtta 360
361 aaacaatcgtttataacgtaaaccaagatactttagacagctctgatgttatcgtttcag 420
421 gtgcttcttgtactacaaactcacttgctccagtagcaaaagttttaaacgacagctttg 480
481 gtttagttgaaggtttcatgactactattcatgcttacactggtgaccaaaatactcaag 540
541 atggtccacacagaaaaggtgacaaacgtcgtgcacgtgcagcagctcaaaacatcgtac 600
601 caaactcaactggtgctgctaaagcaatcggtaaagtaatccctgaaattgacggtaaat 660
661 tagacggtggtgctcaacgtgttcctgttgcaactggttcattaacagaattaacagttg 720
721 tattagacaaagaagtttcagttgacgaagtaaacgaagcaatgaaacaagcttctaacg 780
781 aatcattcggttacaatgaagacgaaatcgtatcttctgacgtggttggtatgacattcg 840
841 gttcattattcgatgctacacaaactcgtgtgatgactgtatcaggtcgtcaattagtta 900
901 aagttgcagcttggtatgataacgaaatgtc (SEQ ID NO:19)
(20) *Staphylococcus saprophyticus* 
1 atggttttggtagaattggtcgtttagcattcagaagaattcaaaacgttgacggaatcg 60
61 acgtagtagcagtaaacgatttaacagatgacgaaatgttagctcatttattaaaatatg 120
121 atactatgcaaggacgcttcacaggagaagttgaagtagaaaaagacggtttccgcgtaa 180
181 acggacaagaagtaaaatcattctctgagcctgaaccaagtaaattaccttggaaagact 240
241 tagacatcgatgttgtattagaatgtactggtttcttcgctgataaagaaaaagcagaag 300
 
301 cacacatcaatgcaggtgctaaaaagtattaatctctgctccagctacaggcgatttaa 360
361 aaacaatcgtttataatacaaaccaccaagaattagacggttcagaaactgttgtttcag 420
421 gtgcttcatgtactactaactcattagctcctgttgctaaagttttaatgatgacttcg 480
481 gtttagtagaaggtttcatgactactatccacgcatacactggtgaccaaagcacactag 540
541 atgcaccacacagaaaaggcgacaaacgtcgtgcgcgtgcagctgctgaaaacatcatcc 600
601 ctaactcaactggtgctgctaaagcaattggcttagtaattcctgaaattgatggtaaat 660
661 tagatggaggagcgcaacgtgttcctgttgcaactggttcattaactgaattaacagttg 720
721 ttttagnnnnnntgtaagcattgaagatgtaaatgctgcaatgaaaaatgcttcaaacg 780
781 aatcattcggttacacagaagacgaaatcgtatcttcagacgtaatcggtatgacttatg 840
841 gttcattatttgatgcaacacaaactcgtgtaatgactgttggcgaccgtcaattagtta 900
901 aagtagcagcttggtatgataacgaaatgtc (SEQ ID NO:20)
(21) *Staphylococcus schleiferi* 
1   atggttttggtagaattggtcgtttagcatttagaagaattcaagatgtagaaacattg 60
61  aggtcgtagctgtaaacgatttaacagatgacgatatgcttgcacatttattgaaatatg 120
121 acacaatgcaaggacgttttactgaagaagtggaagtaattgatggtggtttccgtgtga 180
181 atggtaaagaagcgaaatcattctctgaaccagaacctgctaaattaccttggggtgacc 240
241 ttggtgtggacgtagtattagaatgtactggtttcttcacagataaagaaaaagctgaag 300
301 cacacattcaagcaggcgctaaaaaagtattaatctcagcaccggctaaaggtgatctta 360
361 aaacaatcgtatataatgttaaccacgacgatttagatggttctgaaacagttgtttcag 420
421 gtgcatcatgtactacaaactcattagcacctgttgcaaaaactttacacgacgaattcg 480
481 gtattgttgaaggtttaatgactacaattcacgcatatactggtgaccaaaatacacaag 540
541 atgcacctcacagaaaaggtgacaaacgtcgtgcgcgtgctgctgctgaaaacattatcc 600
601 ctaactctacaggggcagcaaaagcaatcggtttagttattccagaaattgccggtaaat 660
661 tagacggtggtgcacaacgtgttccagttgcaactggttcattaacagaattaacagttg 720
721 ttttagataaagaagtgactgttgaagaagtaaacaaagtattgaaagcagcaactaacg 780
781 aaccattcggttacactgaagacgaaattgtttcttcagacgttgtaggtatgacttacg 840
841 gtgcattattcgatgcaactcaaactcgtgtaatgactgttggcgaccgtcaattagtta 900
901 aagttgcagcttggtatgataacgaaatgtc (SEQ ID NO:21)
(22) *Staphylococcus sciuri* 
1 atggttttggtagaattggtcgtttagcatttagaagattacaagaagttgaaaatatcg 60
61 aagtagtagcaatcaacgatttaacagatgacgcaatgttagctcatttattaaaatatg 120
121 attcaacacaaggtcgtttcaaagacgaagtagaagttatcgaaggcggattccgcgtaa 180
181 acggtagagaaatcaaaactttcgaaaatcctaatcctaaagaattaccatggggcgatt 240
241 tagatatcgatgtagtattagaatgtactggtttcttcgctgacaaagacaaagcttcag 300
301 ctcacatcgaagcaggtgctaaaaaagtattaatttcagctccagcatcaggtgacttaa 360
361 aaactatcgtttataacgttaaccatgacgaattagacggatctgaagaaatcgtttcag 420
421 gtgcatcttgtacaactaactgtttagctccaatggctaaagtattaaatgatgaattcg 480
481 gtatcgttgaaggtttaatgatgacaattcacgcttacactggtgaccaaaatactttag 540
541 atgctccacatgctaaaggtgacttccgtcgtgctcgtgcagctgctcaaaacatcgtgc 600
601 ctaactcaactggtgctgctaaagcaatcggtttagtaattccagaattaaaggtaaat 660
 
661 tagatggatcagctcaacgtgttccagtagcaactggttcagtaacagaattaacagctg 720
721 tattggacaaagaagtttcagttgaagaaatcaatgcagcaatgaaaaatgctacaaatg 780
781 attcattcggttacactgaagacgaaatcgtatcttctgatatcattggtatcacttacg 840
841 gttcattatttgatgcaactcaaactcgtgttatgacagttggagatcgccaattagtta 900
901 aaactgcagcttggtatgataacgaaatgtc (SEQ ID NO:22)
(23) *Staphylococcus simulans* 
1 atggttttggtagaattggtcgtttagcatttcagaagaattcaagatgttgaaggtatc 60
61 gatgtagtagcagtaaacgacttaacagatgatgaaatgttagcacacttattaaaatac 120
121 gatacaatgcaaggacgtttcactgaagaagttgaagttgtagatggcggattccgcgtg 180
181 aatggtaaagaagttaaatcattcgaagaaccagatgcaagcaaattaccttggaaagat 240
241 ttagacattgatgtcgtattagaatgtactggtttctacactagcgacgaaaaagcacaa 300
301 gctcacattgacgcaggtgctaaaaaagtattaatctctgcaccagcaactggtgacgtt 360
361 aaaacaatcgtttataacgtaaaccaagatactttagacagctctgacgtaatcgtttca 420
421 ggtgcttcttgtactacaaactcacttgctccagtagcaaaagtattaaatgacagcttc 480
481 ggtttagtagaaggtttcatgactactatccacgcttacactggtgaccaaaatactcaa 540
541 gacggtccacacagaaaaggcgacaaacgtcgtgcacgtgcagcagctgaaaacatcgtt 600
601 cctaactcaactggtgctgctaaagcaatcggtaaagtaattcctgaaatcgacggaaaa 660
661 ttagacggtggcgctcaacgtgttcctgtagcaactggttcattaactgaattaacagtt 720
721 gtattagacaaagacgtaacaatcgaagaagtaaacgaagctatggaagcagcttctaac 780
781 gaatcattcggttacaacgaagacgaaatcgtatcttcagacgtagttggtatgacattc 840
841 ggttcattattcgatgcaactcaaactcgtgttatgactgtatctggtcgtcaattagtt 900
901 aaagttgcagcttggtatgataacgaaatgtc (SEQ ID NO:23)
(24) *Staphylococcus xylosus* 
1 atggttttggtagaattggtcgtttagcatttagaagaattcaaaacgttgacggaattg 60
61 acgtagtagcagtaaatgacttaacagatgacgaaatgttagcacatttattaaaatatg 120
121 acactatgcaaggacgcttcacaggagaagttgaagtagaaaatgacggtttccgtgtaa 180
181 acggacaagaagtaaaatcattctctgagccagacccaagcaaattaccttggaaagatt 240
241 tagacatcgatgttgtattagaatgtactggtttctacgctgataaagaaaaagcagaag 300
301 ctcacattaatgcaggtgctaaaaaagtattaatttcagctccagctactggtgatttaa 360
361 aaacaatcgtttataacacaaaccaccaagagttagatggtaaagaaacagttgtttcag 420
421 gtgcttcatgtacaactaactcattagctccagttgctaaagtattaaatgatgactttg 480
481 gtttagtagaaggtttcatgactacaattcatgcttacactggtgaccaaaatacacaag 540
541 atgcgccacacgctaaaggcgacaaacgtcgtgctcgtgcagctgctgaaaacattatcc 600
601 ctaactcaactggtgctgctaaagcaattggcctagttatccctgaaattgatggtaaat 660
661 tagacggtggagcgcaacgtgttcctgtagctactggttcgttaacagaattaacagttg 720
721 tattagacaaaaatgtaagtgttgaagacgttaatgctgcaatgaagaatgcttcaaacg 780
781 aatcattcggttacactgaagacgaaatcgtttcttctgatgtagttggtatgacttacg 840
841 gttcattattcgatgcaacacaaacacgtgttatgacagttggcgatcgccaattagtta 900
901 aagtagcagcttggtatgataacgaaatgtc (SEQ ID NO:24)
(25) *Staphylococcus caseolyticus* 
1 atggttttggtagaattggtcgtttattagaacagcatgcacggtaaaagcattggatg 60
61 cacaatatccagaaattgatatttattcaggtgttgagatggatatattagcagatggcg 120
121 aaatggattatagcaatgacgtgctagcacagcttgactattgtattggtgctattcatc 180
181 agtcgttgaatcaaagtgaagatgagattatgaaacgcctgatcaatgcctgtaataatc 240
241 catacattagacatattgctcatccaacgggaaggttgattggtcgccgtaatggttatc 300
301 atgtaaacatgccgaaactcatcgaaactgcacagaagacaaataccatccttgaaatca 360
361 atgcacatccgatgcgccttgatttatcgagcgacgtattaaagcaatatccagatatta 420
421 aacttgtgatcaacacagacgcgcgtgcaatcgatcagcttgatttaatgaaatatggtg 480
481 tgggtacagcaataaaggccatgtgaaaaagaacaggtaataaacactttaccgcgta 540
541 aggattttaaatcttggatacagaatggaagtaattatatgaataaaaagcattaaat 600
601 atattagaatataacaaaattattgagcgtgttgacgcatttactcaaatgaactttca 660
661 agcaaaaagtgaggatgacacancctatcagcgacaaagctgaaatagatagcatgcttg 720
721 cacagcttggtatgataacgaaatgtc (SEQ ID NO:25)
(26) *Staphylococcus aureus* 
1 atggttttggtagaattggtcgtttagcattcagaagaattcaagaagtagaaggtcttg 60
61 aagttgtagcagtaaacgacttaacagatgacgacatgttagcgcatttattaaaatatg 120
121 acactatgcaaggtcgtttcacaggtgaagtagaggtagttgatggtggtttccgcgtaa 180
181 atggtaaagaagttaaatcattcagtgaaccagatgcaagcaaattaccttggaaagact 240
241 taaatatcgatgtagtgttagaatgtactggtttctacactgataaagataaagcacaag 300
301 ctcatattgaagcaggcgctaaaaaagtattaatctcagcaccagctactggtgacttaa 360
361 aaacaatcgtattcaacactaaccaccaagagttagacggttctgaaacagttgtttcag 420
421 gtgcttcatgtactacaaactcattagcaccagttgctaaagttttaaacgatgactttg 480
481 gtttagttgaaggtttaatgactacaattcacgcttatacaggtgatcaaaatacacaag 540
541 acgcacctcacagaaaaggtgacaaacgtcgtgctcgtgcagcagcagaaaacatcatcc 600
601 ctaactcaacaggtgctgctaaagctatcggtaaagttattcctgaaatcgatggtaaat 660
661 tagatggtggtgcacaacgtgttcctgtagctacaggttcattaactgaattaacagtag 720
721 tattagaaaaacaagacgtaacagttgaacaagttaacgaagctatgaaaaatgcttcaa 780
781 acgaatcattcggttacactgaagacgaaatcgtttcttcagacgttgtaggtatgactt 840
841 acggttcattatttgacgctacacaaactcgtgtaatgtcagttggcgaccgtcaattag 900
901 ttaaagttgcagcttggtatgataacgaaatgtc (SEQ ID NO:26)
(27) *Staphylococcus warneri*

1 atggttttggtagaattggtcgtttagcatttagaagaattcaagacgtagaaggtttag 60
61 aagtagttgcagtaaacgacttaactgatgacgatatgttagcacacttattaaaatatg 120
121 acactatgcaaggtcgcttcactggtgaagtagaagttatcgacggtggattccgtgtta 180
181 acggtaaggaagttaaatcattcgatgaaccagatgcaagcaaattaccttggaaagact 240
241 tagatatcgacgtagtattagaatgtactggtttctatactgacaaagataaagcacaag 300
301 ctcatgatgacgcaggtgctaaaaagtattaatctcagctccagcaactggtgacttaa 360
361 aaacaatcgtttacaacactaaccacgatgaattagatggttctgaaacagtagtatcag 420
421 gtgcatcatgtactactaactcattagctcctgtagctaaagtgttaagtgatgagttcg 480

481 gtttagttgaaggtttaatgacaactatccacgcttacactggtgaccaaatactcaag 540
541 atgctccacacagaaaaggcgacaaacgtcgtgctcgtgctgcagcagaaacatcatcc 600
601 ctaactcaactggtgctgctaaagcaatcggtaaagtaattcctgaaatcgacggaaaat 660
661 tagacggtggtgcacaacgtgttccagttgcaactggttcattaactgaattaacagtag 720
721 tattagaaaaagacgtaacagttgaacaagttaacgaagcaatgaaacaagcttctgacg 780
781 aatcattcggttacactgaagacgaaatcgtatcttctgatgtagttggtatgacttacg 840
841 gttcattattcgatgcaactcaaactcgtgtaatggctgttggtggtcgtcaattagtta 900
901 aagtagcagcttggtatgataacgaaatgtc (SEQ ID NO:27)

SEQ ID Nos:1-25 correspond to NCBI GenBank Accession Nos. AF495474-495498, respectively; SEQ ID NO:26 corresponds to nucleotides 1867-2800 of NCBI GenBank Accession No. AJ133520; and SEQ ID NO:27 corresponds to nucleotides 32-962 of NCBI GenBank Accession No. AY024363.

The present invention also provides a method for detecting *Staphylococcus* spp. Specifically, a nucleic acid template from a sample suspected of containing *Staphylococcus* spp. is amplified with a pair of *Staphylococcus* spp.-specific primers. The amplification product, if any, is detected by either gel electrophoresis and staining, or by probe hybridization. Detection of an expected amplification product indicates the presence of *Staphylococcus* spp. in the sample.

The nucleic acid template can be DNA (e.g., a genomic fragment or a restriction fragment) or RNA, in a purified or unpurified form. A nucleic acid template can be obtained from a human or an animal (e.g., a specimen).

The present invention features *Staphylococcus* spp.-specific primers containing oligonucleotides selected from the *gap* gene region described above. One primer contains an oligonucleotide selected from a member of the group consisting of SEQ ID NOs:28-52 (corresponding to SEQ ID NOs:1-25 but excluding the first 26 and the last 25 nucleotides); the other primer contains an oligonucleotide selected from a sequence complementary to the member. Typically, a primer is 14-40 nucleotides in length (PCR Application Manual, Boehringer Mannheim, 1995, page 37). Non-*Staphylococcus* sequences can be added to the 5'-end of a primer. An example of a non-*Staphylococcus* sequence is a sequence containing a restriction site, which can be used to facilitate cloning of the amplification product.

The present invention also features *Staphylococcus*-specific probes chosen from the *gap* gene region described above, i.e., SEQ ID NOs:1-27 and their complimentary sequences. These probes can be used for detecting *Staphylococcus* spp. by hybridizing to an unamplified *Staphylococcus* nucleic acid or an *Staphylococcus* nucleic acid amplified with the above-described primer pairs. SEQ ID NOs:1-27 and their complimentary sequences are examples of such probes.

The probes can be immobilized on the surface of a solid support, such as a membrane (a nylon membrane or a nitrocellulose membrane), a glass, or a plastic polymer. Immobilization of probes to a membrane can be achieved by baking at 80°C or UV crosslinking. The probes can also be covalently linked to a material (e.g., poly-lysine) coated on the surface of a glass. In addition, a novel method of immobilizing probes on a plastic polymer has recently been developed. See US Application Serial No. 09/906,207. Alternatively, the probes can be synthesized *de novo* at precise positions on a solid substrate. See Schena *et al*., 1995, *Science* 270: 467; Kozal *et al*., 1996, *Nature Medicine* 2(7): 753; Cheng *et al*., 1996, *Nucleic Acids Res.* 24(2): 380; Lipshutz *et al*., 1995, *BioTechniques* 19(3): 442; Pease *et al*., 1994, *Proc. Natl. Acad. Sci. USA* 91: 5022; Fodor *et al*., 1993, *Nature* 364: 555; and Fodor *et al*., WO 92/10092.

A target *Staphylococcus* nucleic acid (e.g., an amplification product described above) can be detected by binding it to an immobilized probe. To facilitate the detection, a labeled amplification product can be generated with a labeled amplification primer. Alternatively, the labeling can be done, chemically or enzymatically, after amplification. Examples of labeling reagents include, but are not limited to, a fluorescent molecule (e.g., fluorescein and rhodamine), a radioactive isotope (e.g., ³²P and ¹²⁵I), a colorimetric reagent, and a chemiluminescent reagent. Biotin and digoxgenin are frequently used for colorimetric detection on a membrane or a plastic polymer. Fluorescent labels, such as Cy3 and Cy5, are widely used for detection on a glass. In addition, artificial tagging tails (e.g., a protein or its antibody) can be conjugated to the 5'-end of the primers or either end of the probes. See Stetsenko and Gait, 2000, *J. Org. Chem.* 65(16): 4900.

The specificity of the *Staphylococcus* spp. detection method of this invention is unexpectedly high. A probe derived from the *gap* gene region of *Staphylococcus intermedius* (67-71% identical to 25 other *Staphylococcus* spp.) detected only genomic DNA from *Staphylococcus* spp., but not that from other bacteria such as *Salmonella* spp., *E. coli, Shigella* spp., *Enterobacter aerogenes, Citrobacter freundii, Klebsiella pneumoniae, Listeria monocytogenes, Vibrio parahaemolyticus, Bacillus cereus,* and *Streptococcus aglactiae* (see Example 2 below). Most unexpected is the ability of a probe derived from a *Staphylococcus* species to discriminate this *Staphylococcus* species from other *Staphylococcus* species having as high as 94% homology in the *gap* gene region.

Also within the scope of this invention is use of *Staphylococcus* spp.-specific sequences described above in combination with other species-specific nucleic acid sequences for simultaneously identification of multiple microorganisms.

The specific examples below are to be construed as merely illustrative, and not limitative of the remainder of the disclosure in any way whatsoever. Without further elaboration, it is believed that one skilled in the art can, based on the description herein, utilize the present invention to its fullest extent

### Example 1: Typing Staphylococcus spp. by probe hybridization

### 1. Bacterial strains

Twenty-seven *Staphlylococci* strains were obtained from Culture Collection and Research Center (CCRC), Hsin-Chu, Taiwan. The strains and their registration numbers are listed in TABLE 1.

**TABLE 1**

| **LIST OF *STAPHYLOCOCCUS* STRAINS** | | | |
|---|---|---|---|
| **No.** | **Bacterial Strain** | **CCRC No.** | **ATCC No.** |
| 1 | *Staphylococcus arlettae* | 13975 | 43957 |
| 2 | *Staphylococcus auricularis* | 13912 | 33753 |
| 3 | *Staphylococcus caprae* | 13911 | 35538 |
| 4 | *Staphylococcus capitis* | 12161 | 27840 |
| 5 | *Staphylococcus carnosus* | 12922 | - |
| 6 | *Staphylococcus chromogenes* | 12924 | 43764 |
| 7 | *Staphylococcus cohni* | 12155 | 29974 |
| 8 | *Staphylococcus delphini* | 15270 | 49171 |
| 9 | *Staphylococcus epidermidis* | 10783 | 155 |
| 10 | *Staphylococcus equorum* | 13974 | 43958 |
| 11 | *Staphylococcus gallinarum* | 13913 | 35539 |
| 12 | *Staphylococcus haemolyticus* | 12923 | 29970 |
| 13 | *Staphylococcus hominis* | 12156 | 27844 |
| 14 | *Staphylococcus hyricus* | 12925 | 11249 |
| 15 | *Staphylococcus intermedius* | 15235 | 49051 |
| 16 | *Staphylococcus kloosii* | 13973 | 43959 |
| 17 | *Staphylococcus lentus* | 12926 | 29070 |
| 18 | *Staphylococcus lugdunesis* | 13971 | 43809 |
| 19 | *Staphylococcus piscifermentans* | 15314 | 51337 |
| 20 | *Staphylococcus saprophyticus* | 10786 | 15305 |
| 21 | *Staphylococcus schleiferi* | 13972 | 43808 |
| 22 | *Staphylococcus sciuri* | 12972 | 29062 |
| 23 | *Staphylococcus simulans* | 10778 | 11631 |
| 24 | *Staphylococcus xylosus* | 12930 | 29971 |
| 25 | *Staphylococcus caseolyticus* | | 13548 |
| 26 | *Staphylococcus aureus* | 10780 | 12600 |
| 27 | *Staphylococcus warneri* | 12929 | 27836 |

### 2. Cultivation of bacterial strains

One loopful of each test strain was plated on Luria-Bertani agar (LB; 0.5% yeast extract, 1% trypton, 0.5% NaCl, 1.5-2% agar) and incubated for overnight (14 hr) at 37°C to recover the bacterial vitality. A single colony was picked for each strain, inoculated into 3 ml sterilized LB broth, and incubated for overnight at 37°C with shaking at 150-180rpm. The cell density of each bacterial strain was up to ∼1x10¹⁰ cells/ml.

### 3. Preparation of bacterial total genomic DNA

Total genomic DNA was prepared from 1ml of bacterial overnight culture. Cells were harvested by centrifugation at 6,000xg for 5 min and discarding the culture supernatant. Cell pellets were resuspended in 50µl STET buffer (0.1M NaCI, 10mM Tris-HCI, pH 8.0, 1mM EDTA, and 5% Triton X-100) containing 5µl lysozyme (10mg/ml). The cells were lysed after incubation for 15min at 37°C followed by 10min-boiling. The DNA-containing supernatant were roughly separated from cell debris after 5-min centrifugation, extracted with phenol/chloroform (1:1), and centrifuged at 10,000xg for 10min. The upper layer of the extraction mixture, ca. 40 µl, was transferred to a new Eppendorf tube and used as a DNA template for amplification.

### 4. Amplification of bacterial genomic DNA with genus-specific oligonucleotide primers

Partial *gap* gene sequences of 27 different *Staphylococcus* species (supra) were analyzed by amplification using a primer pair (Yugueros *et al*., 2000, *J. Clinical Microbiology* 38: 4351-4355) located in the conserved region of the *gap* gene and subsequent sequence analysis. The primers used were GF-1(5'-atggttttggtagaattggtcgttta-3') and GR-2(5'-gacatttcgttatcataccaagctg-3'), both of which were synthesized by GENASIA SCIENTIFICS INC. This pair of primers has been shown to be specific for *Staphylococcus* (Yugueros *et al.*, 2000, *J. Clinical Microbiology* 38: 4351-4355). The amplification reaction mixture (50 µl) contained 5 µl 10X Taq DNA polymerase buffer (Promega, Madison, WI, USA), 5 µl 25mM MgCl₂ (Promega), 5µl 2.5mM dNTPs (Promega), 1µl 20µM of each oligonucleotide primer, 1 µl of extracted total genomic DNA, 1 U of Taq DNA polymerase (Promega), and sterilized dH₂O. Amplification was carried out using GeneAmp® PCR System 2400 (Perkin-Elmer) as followed: 95°C for 2min; 30 cycles of 95°C for 30 sec, 55°C for 30 sec, 72°C for 30 sec; and a final extension of 72°C for 6 min.

### 5. Cloning and sequencing of Staphylococcus spp. partial gap gene fragments

Each of the amplified partial *gap* gene fragments (ca. 933 bp) was cloned into a pGEM-T Easy vector system (Promega, Madison, WI, USA), which was transformed into an *E. coli* bacterial host. Three *E. coli* transformants were selected from each transformation, and the plasmid DNA containing the amplified *Staphylococcus* spp. *gap* gene fragment was isolated using QIAamp DNA Mini Kit (Qiagen, Hilden, Germany). The presence of the amplified partial *gap* gene fragment was confirmed first by restriction enzyme digestion and then by sequencing using two primers, T7 promoter primer and SP6 promoter primer (Promega, Madison, WI, USA). Twenty-five of these gene fragments are newly identified sequences; 2 of them have been previously published (i.e., *Staphylococcus aureus gap* gene fragment, NCBI GenBank Accession No. AJ133520, nucleotides 1867-2800; and *Staphylococcus warneri gap* gene fragment, NCBI GenBank Accession No. AY024363, nucleotides 32-962).

The plasmids containing the 27 *Staphylococcus* spp. partial *gap* gene fragments are listed in TABLE 2 below.

**TABLE 2**

| **PLASMIDS CARRYING PARTIAL** **STAPHYLOCOCCUS SPP. GAP GENE FRAGMENTS** | | |
|---|---|---|
| **No.** | **Strains** | **Plasmid Name** |
| 1 | *Staphylococcus aureus* | pGAP-A1-1 |
| 2 | *Staphylococcus arlettae* | pGAP-A1-34 |
| 3 | *Staphylococcus auricularia* | pGAP-A2-2 |
| 4 | *Staphylococcus carnusus* | pGAP-A2-5 |
| 5 | *Staphylococcus caprae* | pGAP-A1-29 |
| 6 | *Staphylococcus capitis* | pGAP-A1-46 |
| 7 | *Staphylococcus caseolyticus* | pGAP-A2-37 |
| 8 | *Staphylococcus hominis* | pGAP-A1-4 |
| 9 | *Staphylococcus cohni* | pGAP-A1-7 |
| 10 | *Staphylococcus chromogenes* | pGAP-A2-31 |
| 11 | *Staphylococcus delphini* | pGAP-A2-19 |
| 12 | *Staphylococcus epidermidis* | pGAP-A1-32 |
| 13 | *Staphylococcus equorum* | pGAP-A2-16 |
| 14 | *Staphylococcus gallinarum* | pGAP-A2-5 |
| 15 | *Staphylococcus haemolyticus* | pGAP-A1-41 |
| 16 | *Staphylococcus hyricus* | pGAP-A2-27 |
| 17 | *Staphylococcus intermedius* | pGAP-A2-38 |
| 18 | *Staphylococcus kloosii* | pGAP-A2-14 |
| 19 | *Staphylococcus lentus* | pGAP-A1-17 |
| 20 | *Staphylococcus lugdunesis* | pGAP-A2-9 |
| 21 | *Staphylococcus piscifermentans* | pGAP-A2-20 |
| 22 | *Staphylococcus saprophyticus* | pGAP-A1-38 |
| 23 | *Staphylococcus sciuri* | pGAP-A1-50 |
| 24 | *Staphylococcus schleifer* | pGAP-A2-11 |
| 25 | *Staphylococcus simulans* | pGAP-A1-22 |
| 26 | *Staphylococcus warneri* | pGAP-A1-19 |
| 27 | *Staphylococcus xylosus* | pGAP-A1-24 |

### 6. Sequence alignment of amplified partial gap gene fragments

Classification of homology degree of these bacteria is based on sequence analysis results obtained by using the phylogenetic tree and sequence pair distance program (Lesegene, DNAstar Inc., Wisconsin, USA) and the BLAST sequence alignment program (NCBI GenBank). The homology degree among 26 of the *Stapkylococcus* spp. is in the range of 68-99%. One exception is the strain *Staphylococcus caseolyticus*, which shows only 22-27% homology to the other 26 *Staphylococcus* spp. This strain has been previously misidentified as *Micrococcus caseolyticus* (Schleifer *et al*., 1982, *J. Systematic Bacteriology* 32: 15-20). DNA-DNA hybridization data and other physiological and biochemical data have been used to reclassify this bacterium. Thirteen pairs of *Staphylococcus* spp. (TABLE 3) have a homology degree over 90%. In particular, pair no. 1 (i.e., *Staphylococcus epidermidis - Staphylococcus warneri*) shows 99% homology, and the other 12 pairs have a homology degree up to 94%.

**TALBE 3**

| **HOMOLOGY DEGREES FOR THIRTEEN PAIRS OF *STAPHYLOCOCCUS* SPP.** | | | |
|---|---|---|---|
| **Pair** **No.** | **Bacterial Strain-1** | **Bacterial Strain-2** | **Homology** **Degree*** |
| 1 | *Staphylococcus epidermidis* | *Staphylococcus warneri* | 99.5 % |
| 2 | *Staphylococcus capitis* | *Staphylococcus caprae* | 94% |
| 3 | *Staphylococcus camusus* | *Staphylococcus hyricus* | 94.3% |
| 4 | *Staphylococcus camusus* | *Staphylococcus simulans* | 93.8% |
| 5 | *Staphylococcus* *saprophyticus* | *Staphylococcus xylosus* | 92.3% |
| 6 | *Staphylococcus lentus* | *Staphylococcus sciuri* | 92.3% |
| 7 | *Staphylococcus wareri* | *Staphylococcus capitis* | 91.3% |
| 8 | *Staphylococcus hyricus* | *Staphylococcus chromogenes* | 91.2% |
| 9 | Staphylococcus epidermidis | Staphylococcus capitis | 91.1% |
| 10 | Staphylococcus saprophyticus | Staphylococcus equorum | 90.5% |
| 11 | Staphylococcus arlettae | Staphylococcus kloosii | 90.5% |
| 12 | Staphylococcus caprae | Staphylococcus wareri | 90.3% |
| 13 | Staphylococcus caprae | Staphylococcus epidermis | 90.1% |

| | | | |
|---|---|---|---|
| *: Degree of homology between two species is determined by using either the DNA sequence analyzing software Lesegene (i.e., pair nos 3-13) or GenBank BLAST (i.e., pair nos 1 and 2). | | | |

### 7. Preparation of hybridization probes and simulative target DNA

Both hybridization probes and simulative target DNA were generated by the amplifying the *gap* gene of three bacteria, *Staphylococcus aureus, Staphylococcus caprae*, and *Staphylococcus capitis*, using the primer pair GF-1 and GR-2. The amplified partial *gap* gene fragments from *Staphylococcus caprae* and from *Staphylococcus capitis* are 94% identical, while each of them shows 89.2% homology to the *Staphylococcus aureus gap* gene fragment.

The amplification products (around 930 bp) obtained by using biotin-labeled primers were used as simulative target DNA, while the amplification products obtained by using unlabeled primers were used as hybridization probes. The amplification was carried out as described above, except that around 100 ng purified plasmid DNA was used as DNA template instead of bacterial genomic DNA. The amplified products were extracted with phenol/chloroform (v/v = 1/1) and precipitated with ethanol to remove excess primers, dNTPs and the enzyme.

### 8. Hybridization

The three hybridization probes were dissolved in a probe solution (DR. Probsol, DR. Chip Biotechnology Inc.) to a final concentration of 20 ng/l, spotted and immobilized on a solid support (DR. Chip Biotechnology Inc.). Four microliters of each biotin-labeled simulative target DNA (stock concentration: 40 ng/.l) were mixed with 500 .1 hybridization buffer (Dr. Hyb™ buffer, DR. Chip Biotechnology Inc.). The DNA mixture was boiled for 5 min, chilled, applied to the solid support, and incubated at 80°C for 1 hr with shaking. Three wash steps were carried out to eliminate unspecific binding. The solid support was first washed with a wash buffer (DR.Wash from DR. Chip Biotechnology Inc., Taiwan) for at least three times at room temperature. A stringent wash was then performed with the same wash buffer at 80°C for 30 min with shaking. The solid support was finally washed with the same wash buffer for at least three times at room temperature again. Biotin-specific colorimetric detection was performed by incubating the solid support in a Blocking Reagent (Roche) containing alkaline phosphatase-conjugated streptavidin (Promega). The solid support was subsequently washed three times with the wash buffer, and incubated with NBT/BCIP solution (Roche) diluted with a detection buffer in a ratio recommended by the supplier for about 10 minutes in dark.

Unexpectedly, the *Staphylococcus aureus* target DNA only hybridized to the probe originated from the same bacterial species, but not to the probes originated from the other two *Staphylococcus* species. Similarly, the *Staphylococcus caprae* and *Staphylococcus capitis* target DNAs only hybridized to the corresponding probe. This result indicates that this method can be used to differentiate *Staphylococcus* spp. strains with very high homology degree (e.g., 94%).

### Example 2: Detecting Staphylococcus genus by probe hybridization

### 1. Bacterial strains

Eighty food-borne bacterial strains listed in TABLE 4 were used in this study. Group I contains 10 *Staphylococcus* spp.; Group II contains another 10 *Staphylococcus* spp.; Group III contains 10 *Salmonella* spp.; Group IV contains 30 *E. coli* strains, including non-pathogenic strains and pathogenic strains such as ETEC, EIEC, EPEC, EAggEC and EHEC; and Group V contains other bacterial strains, including 11 pathogenic strains (i.e., 4 *Shigella* spp.; 3 coliform bacterial strains *Enterobacter aerogenes, Citrobacter freundii*, and *Klebsiella pneumoniae*; 3 other food-borne pathogenic bacterial strains *Listeria monocytogenes, Vibrio parahaemolyticus*, and *Bacillus cereus;* and 1 infectious bacterial strain causing cattle mastitis, *Streptococcus agalactiae*). These bacterial strains were obtained from different sources, i.e., Culture Collection and Research Center (CCRC), Hsin-Chu, Taiwan; American Type Culture Collection (ATCC), Rockville, MD, USA; United States Department of Agriculture (USDA), Washington, DC, USA; Department of Food Science, National Chung-Hsing University (NCHU; Taichung, Taiwan, R.O.C.); Pingtung University of Technology (PT), Pingtung, Taiwan; and Deutsche Sammlung von Mikroorganismen und Zellkulturen GmbH (DSMZ; Braunschweig, Germany).

**TABLE 4**

| **BACTERIAL STRAINS** | | | | |
|---|---|---|---|---|
| **Group** | **Strains** | **No.** | **Source & Number** | |
| Group I *Staphylococcus* | *Staphylococcus arlettae* | 1 | CCRC 13975 | ATCC 43957 |
| | *Staphylococcus auricularia* | 1 | CCRC 13912 | ATCC 33753 |
| | *Staphylococcus chromogenes* | 1 | CCRC 12924 | ATCC 43764 |
| | *Staphylococcus epidermidis* | 1 | CCRC 10783 | ATCC 155 |
| | *Staphylococcus gallinarum* | 1 | CCRC 13913 | ATCC 35539 |
| | *Staphylococcus hominis* | 1 | CCRC 12156 | ATCC 27844 |
| | *Staphylococcus lentus* | 1 | CCRC 12926 | ATCC 29070 |
| | *Staphylococcus piscifermentans* | 1 | CCRC 15314 | ATCC 51337 |
| | *Staphylococcus simulans* | 1 | CCRC 10778 | ATCC 11631 |
| | *Staphylococcus schleiferi* | 1 | CCRC 13972 | ATCC 43808 |
| | *Staphylococcus xylosus* | 1 | CCRC 12930 | ATCC 29971 |
| | *Staphylococcus capitis* | 1 | CCRC 12161 | ATCC 49324 |
| | *Staphylococcus carnusus* | 1 | CCRC 12922 | DSM 20501 |
| Group II *Staphylococcus* | *Staphylococcus cohni* | 1 | CCRC 12155 | ATCC 29974 |
| | *Staphylococcus equorum* | 1 | CCRC 13974 | ATCC 43958 |
| | *Staphylococcus haemolyticus* | 1 | CCRC 12923 | ATCC 29970 |
| | *Staphylococcus hyricus* | 1 | CCRC 12925 | ATCC 11249 |
| | *Staphylococcus kloosii* | 1 | CCRC 13973 | ATCC 43959 |
| | *Staphylococcus lugdunesis* | 1 | CCRC 13971 | ATCC 43809 |
| | *Staphylococcus saprophyticus* | 1 | CCRC 10786 | ATCC 15305 |
| | *Staphylococcus sciuri* | 1 | CCRC 12927 | ATCC 29062 |
| | *Staphylococcus warneri* | 1 | CCRC 12929 | ATCC 27836 |
| | *Staphylococcus delphini* | 1 | CCRC 15270 | ATCC 49171 |
| | *Staphylococcus caseolyticus* | 1 | CCRC 15268 | ATCC 13548 |
| Group III *Salmonella* spp. | *Salmonella typhi* | 1 | CCRC 14875 | ATCC 9992V |
| | *Salmonella typhiurium* | 1 | CCRC 10747 | ATCC 14028 |
| | *Salmonella salamae* | 1 | CCRC 15450 | ATCC 6956 |
| | *Salmonella paratyphi A* | 1 | CCRC 14878 | ATCC 9150 |
| | *Salmonella California* | 1 | CCRC 15454 | ATCC 23201 |
| | *Salmonella enteritidis* | 1 | CCRC 10744 | ATCC 13076 |
| | *Salmonella etterbeele* | 1 | CCRC 15455 | ATCC 19128 |
| | *Salmonella postsdam* | I | CCRC 15453 | ATCC 15782 |
| | *Salmonella aberdeen* | 1 | NCHU | USDA |
| | *Salmonella albany* | 1 | NCHU | USDA |
| | *Salmonella amger* | 1 | NCHU | USDA |
| | *Salmonella anatum* | 1 | NCHU | PT |
| Group IV *E. coli* | EAggEC | 4 | NCHU | - |
| | ETEC | 4 | NCHU | - |
| | EHEC | 5 | NCHU | - |
| | EPEC | 5 | NCHU | - |
| | EIEC | 3 | NCHU | - |
| | Non-pathogenic | 9 | NCHU | - |
| Group V Other strains | *Streptococcus agalactea* | 1 | CCRC10787 | ATCC13813 |
| | *Bacillus cereus* | 1 | CCRC11827 | ATCC25428 |
| | *Shigella dysenteria* | 1 | CCRC13983 | ATCC13313 |
| | *Shigella boydii* | 1 | CCRC15961 | ATCC8704 |
| | *Shigella flexneri* | 1 | CCRC10772 | ATCC12022 |
| | *Shigella sonnei* | 1 | CCRC10773 | ATCC9290 |
| | *Vibrio parahaemolyticus* | 1 | CCRC10806 | ATCC17802 |
| | *Listeria monocytogenes* | 1 | CCRC14930 | - |
| | *Enterobacter aerogenes* | 1 | CCRC10370 | ATCC13048 |
| | *Citrobacter freundii* | 1 | CCRC 12291 | ATCC8090 |
| | *Klebsiella pneumoniae* | 1 | CCRC15627 | - |
| Positive control strains | *Staphylococcus aureus* | 1 | CCRC10780 | ATCC12600 |
| | *Staphylococcus caprae* | 1 | CCRC13911 | ATCC35538 |
| | *Staphylococcus intermedius* | 1 | CCRC15235 | ATCC49051 |

### 2. Cultivation of bacterial strains

One loop of each test strain was plated on Luria-Bertani agar (LB; 0.5% yeast extract, 1% trypton, 0.5% NaCl, 1.5-2% agar) and incubated for overnight (14 hr) at 37°C. A single colony was picked for each strain and inoculated into 10 ml sterilized LB broth. Bacterial cultures were incubated for overnight at 37°C with shaking at 120-150rpm.

### 3. Preparation of bacterial mixtures and extraction of genomic DNA

One milliliter suspension (10⁸-10¹⁰ cells) was taken from each bacterial culture and mixed in one tube according to the groups listed in TABLE 1. QIAamp DNA Mini Kit (Qiagen, Hilden, Germany) was used for extraction of total genomic DNA from 1 ml mixed culture and for further purification. Quantification of extracted genomic DNA was carried out by spectrophotometric method (V-530, Jasco, Jascon International Co., LTD, Japan). The final concentration of the genomic DNA prepared from each bacterial mixture was adjusted to 5µg/µl.

### 4. Immobilization of DNA on a solid support

One microliter (5µg/µl) of prepared DNA (equivalent to a DNA extract from (3.3x10⁶/8) 4.1x10⁵-1.7x10⁸ (10⁹/6) cells for each bacterial strain), including mixed genomic DNA of each bacterial group, genomic DNA of a certain bacteria species, and control plasmid DNA, was dissolved in a probe solution (DR. Probsol, DR.Chip Biotechnology Inc., Taiwan), denatured at 95°C for 5 min, and chilled immediately on ice. The pretreated DNA solution was dotted and cross-linked on a nylon membrane (Hybond™-N, Amersham Pharmacia Biotech) of 5cm X 5cm.

### 5. Preparation of biotin-labeled detection probes

Biotin-labeled probes were generated as described above. Plasmid pGAP-A2-38 containing the *Staphylococcus intermedius gap* gene fragment was used as a DNA template for amplification. Among the 26 *Staphylococcus* spp. (*Staphylococcus caseolyticus excluded*), *Staphylococcus intermedius* shows the lowest homology (67% - 71%) to other species.

### 6. Hybridization

The biotin-labeled detection probe was dissolved in 50ml hybridization buffer (Dr. Hyb™ buffer, DR. Chip Biotechnology Inc.) to a final concentration of 0.1 nM, denatured at 95°C for 5 min, and immediately chilled on ice for 5 min. This pretreated detection probe solution was then hybridized to DNA spotted on a nylon membrane for 12 hr at 50°C. The nylon membrane was then washed with 50 ml wash buffer (DR.Wash from DR. Chip Biotechnology Inc., Taiwan) twice at 50°C for 5 min.

Unexpectedly, positive signals were detected at spots containing the mixed genomic DNA of Group I and II bacteria, the genomic DNA of three *Staphylococcus* spp. (i.e., *Staphylococcus aureus, Staphylococcus caprae*, and *Staphylococcus intermedius*), and positive controls. No signal was detected at spots containing the mixed genomic DNA of Group III, IV, or V bacteria. This result indicates that the *Staphylococcus* spp. *gap* gene fragment can be used to differentiate *Staphylococcus* from other bacterial genera.

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features. From the above description, one skilled in the art can easily ascertain the essential characteristics of the present invention, and without departing from the spirit and scope thereof, can make various changes and modifications of the invention to adapt it to various usages and conditions. Thus, other embodiments are also within the scope of the following claims.

## Claims

1. A nucleic acid comprising an oligonucleotide selected from a member of the group consisting of SEQ ID NOs:1-25 and sequences complementary to SEQ ID NOs:1-25, wherein the nucleic acid is 10-1000 nucleotides in length.

2. The nucleic acid of claim 1, wherein the nucleic acid is 10-500, 10-200, 10-50 or 10-20 nucleotides in length.

3. The nucleic acid of claim 1, wherein the oligonucleotide is a member of the group consisting of SEQ ID NOs:1-25 and sequences complementary to SEQ ID NOs:1-25.

4. A pair of amplification primers, comprising
a first primer containing a first oligonucleotide selected from a member of the group consisting of SEQ ID NOs:28-52, and
a second primer containing a second oligonucleotide selected from a sequence complementary to the member,
wherein each primer is 14-40, 14-30 and/or 14-20 nucleotides in length.

5. A method of detecting a target *Staphylococcus* species, comprising:
providing a sample;
amplifying a nucleic acid contained therein with a pair of primers according to claim 4; and
detecting an amplification product;
whereby detection of the amplification product indicates the presence of the target *Staphylococcus* species.

6. The method of claim 5, wherein the detecting step includes hybridizing the amplification product to a nucleic acid probe that is 10-1000, 10-500, 10-200, 10-50 and/or 10-20 nucleotides in length and contains a sequence selected from a member of the group consisting of SEQ ID NOs:1-27 and sequences complementary to SEQ ID NOs:1-27.

7. The method of claim 6, wherein the nucleic acid probe is a member of the group consisting of SEQ ID NOs:1-27 and sequences complementary to SEQ ID NOs:1-27.

8. A kit for detecting a target *Staphylococcus* species in a sample, comprising a nucleic acid according to any of the claims 1 to 3 and/or amplification primers according to claim 4.


SEQUENCE LISTING

<110>  DR. Chip Biotechnology Incorporation

<120>  Detection of Staphylococcus spp.

<130>  51182 EP

<140>  EP 02027284.5
<141>  2002-12-06

<150>  US 10/164,758
<151>  2002-06-07

<160>  56

<170>  PatentIn version 3.1

<210>  1
<211>  931
<212>  DNA
<213>  Staphylococcus arlettae

<400>  1
atggttttgg tagaattggt cgtttagcat ttagaagaat tcaaaacgtt gatggaatcg      60

acgttgtagc agtaaacgac ttaacagatg atgaaatgtt agcacactta ttaaaatatg     120

acactatgca aggacgtttc acaggagaag ttgaagtaga aaatgacggt ttccgtgtaa     180

atggtcaaga agttaaatca ttctcagaac cagatccaaa taaattacca tggggcgact     240

tagatatcga tgttgtttta gaacgtactg gtttattcgc agacaaagat aaagcatcag     300

ctcatatcga cgcaggtgcg aaaaaagtat taatctctgc tccagcatca ggtgatttaa     360

aaacaatcgt attcaacact aaccacaatg aattagatgg ttctgaaaca gttgtatcag     420

gtgcttcatg tactactaac tcattagctc cagttgctaa agtattaaac gatgacttcg     480

gtttagtaga aggtttaatg actactatcc atgcttacac tggtgaccaa agcacacaag     540

acgctcctca cagaaaaggt gacaaacgtc gtgcgcgtgc agctgctgaa aacattattc     600

ctaactcaac aggtgctgct aaagcaatcg gtttagttat tcctgaaatc gatggtaaat     660

tagatggtgg cgctcaacgt gtaccagtag ctactggttc attaactgaa ttaacagttg     720

tattagacaa agaagtaact gttgaagacg ttaatagtgc aatgaaaaat gcttcaaacg     780

aatcatttgg ttacactgaa gacgaaatcg tttcttctga cgtaatcggt atgacttacg     840

gttcattatt cgatgctaca caaactcgtg taatgactgt tggtaaccaa caaatggtta     900

aagtagcagc ttggtatgat aacgaaatgt c                                    931

<210>  2
<211>  931
<212>  DNA
<213>  Staphylococcus auricularia

<400>  2

atggttttgg tagaattggt cgtttagcat tcagaagaat tcaaaatgtt gaaggcattg 60

acgtagtagc agttaatgac ttaacagatg acgatatgtt agcacactta ttaaaatacg 120

acacaatgca aggtcgtttc acaagtgaag ttgaaatcgt agaaggcggt ttccgcgtta 180

acggtcaaga agtgaaatca ttcgacgaac cagatccaag caaattacct tggaaagact 240

tagacatcga cgttgtactt gaatgtactg gtttattcac agacaaagaa aaagcagaag 300

cacacatcga cgcaggtgct aaaaaagtat taatctctgc accagctaaa ggtgacgtta 360

aaacagttgt ttataacact aaccacgata cacttgatgg tacagaaaca gttgtttcag 420

gtgcttcatg tacaacaaac tcattagcac cagttgctaa agtacttaac gatgaattcg 480

gtttagtaga aggattcatg actacaatcc acgcatacac tggtgaccaa aatacacaag 540

atgcaccaca caaaaaaggt gacaaacgcc gtgcacgtgc agctgctcaa aacatcatcc 600

ctaactctac aggtgctgca aaagcaatcg gtaaagttat cccagaaatc gacggtaaat 660

tagacggcgg tgcacaacgt gtccctgttg ctactggttc attaacagaa ttaacagtag 720

tattagaaaa agatgtttca atcgaagacg taaacaacgc aatgaaaaat gcatcaactg 780

aatcattcgg ttacacagat gacgaaatcg tttcttctga cgtcatcggt atgacttatg 840

gttcattatt cgacgcaact caaactcgtg tcatgactgt tggcgatcac caattaatca 900

aagttgcagc ttggtatgat aacgaaatgt c 931

<210> 3
<211> 934
<212> DNA
<213> Staphylococcus caprae

<400> 3
atggttttgg tagaattggt cgtttagcat tcagaagaat ccaagatgta gaaggtctta 60

aagtagtagc agttaacgac ttaacagacg atgaaatgtt agctcattta ttaaaatatg 120

acactatgca aggacgcttc actggagaag ttgaagttat cgaaggcgga ttccgcgtaa 180

acggtaaaga aattaaatca ttcgacgaac cagatgcaaa caaattacct tggggtgacc 240

ttgacatcga tgtagtatta gaatgtactg gtttctacac tgataaagat aaagcacaag 300

cacatatcga tgcaggtgct aaaaaagtat taatctcagc tccagctact ggtgatttaa 360

aaacaatcgt attcaatact aaccatgacg aattagatgg ttcagaaaca gttgtatcag 420

gtgcttcttg tacaactaac tcattagctc ctgttgctaa agtattaaat gatgaattcg 480

gtttagttga aggtttaatg acaactatcc acgcttacac aggtgaccaa aatactcaag 540

acgcacctca cagaaaaggt gacaaacgtc gtgctcgtgc agcagcagaa aacattatcc 600

ctaactcaac aggtgctgct aaagcaatcg gtaaagtaat tcctgaaatc gacggtaaat 660

tagacggtgg tgcacaacgt gttccagttg ctacaggttc attaacagaa ttaactgtag 720

tattagaaaa acaagatgta actgctgatc aagttaacga agcaatgaaa caagcttctg 780

acgaatcatt cggttacact gaagatgaaa tcgtatcttc tgacgttgta ggtatcactt 840

atggttcatt attcgatgca actcaaactc gtgtaatgac tgttggtgac cgtcaattag 900

ttaaagtagc agcttggtat gataacgaaa tgtc 934

<210> 4
<211> 934
<212> DNA
<213> Staphylococcus capitis

<400> 4
atggttttgg tagaattggt cgtttagcat tcagaagaat ccaagatgta gaaggtctag 60

aagtagttgc agttaacgac ttaacagatg atgaaatgtt agctcactta ttaaaatatg 120

acactatgca aggtcgcttc actggagaag ttgaagtcat cgatggtgga ttccgtgtta 180

acggtaaaga aattaaatca ttcgatgaac cagatgcaag caaattacct tggggagacc 240

tagacatcga cgtagtatta gaatgtactg gtttctacac tgacaaagat aaagcacaag 300

ctcacatcga tgcaggtgct aaaaaagtat taatctctgc tccagctaca ggtgatttaa 360

aaacaatcgt attcaacact aaccatgatg aattagatgg ttctgaaaca gttgtatcag 420

gtgcttcttg tacaactaac tcattagcac cagttgctaa agttttaaat gatgagtttg 480

gtttagttga aggtttaatg actactatcc acgcttatac tggtgaccaa aatactcaag 540

atgctcctca cagaaaaggc gacaaacgtc gtgctcgtgc agcagcagaa aacattatcc 600

ctaactctac aggtgctgct aaagcaattg gtaaagttat tccagaaatc gatggtaaat 660

tagacggtgg agctcaacgt gttccagttg caacaggttc attaactgaa ttaacagtag 720

tattagataa acaagatgta acagctgatc aagtaaatga agcaatgaaa caagcttctg 780

acgaatcatt cggttacact gaagatgaaa tcgtaccttc tgacgttgta ggtatgactt 840

atggttcatt attcgacgct actcaaactc gtgtaatgac tgttggtgac cgtcaattag 900

ttaaagttgc agcttggtat gataacgaaa tgtc 934

<210> 5
<211> 931
<212> DNA
<213> Staphylococcus carnosus

<400> 5
atggttttgg tagaattggt cgtttagcat ttagaagaat tcaagatgtt gaaggtatcg 60

atgtagtagc agtaaacgac ttaacagatg atgaaatgtt agcacactta ttaaaatacg 120

atacaatgca aggacgtttc actgaagaag ttgaagttgt agatggcgga ttccgcgtga 180

atggtaaaga agttaaatca ttcgaagaac cagatgcaag caaattacct tggaaagatt 240

tagacattga tgttgtatta gaatgtacag gtttctatac aagtgaagaa aaagcaaaag 300

ctcatatcga tgcaggcgct aaaaaagtat taatttcagc accagctact ggtgatgtta 360

aaacaatcgt ttataacgta aaccaagata ctttagacag ctctgacgta atcgtttcag 420

gtgcttcttg tactacaaac tctcttgctc ctgtagcaca agttttaaat gacagctttg 480

gtttagttga aggtttcatg actactatcc atgcttatac tggtgaccaa aatactcaag 540

atggtccaca cagaaaaggt gacaaacgtc gtgctcgtgc agcagctgaa aacatcgttc 600

caaactcaac tggtgcagct aaagcaattg gtaaagtaat tcctgaaatc gacggaaaat 660

tagacagcgg cgctcaacgt gttcctgttg caactggttc attagctgaa ttaacagttg 720

tattagataa agacgttaca gttgacgaag taaacgaagc aatgaaacaa gcttctaacg 780

aatcattcgg ttacaacgaa gacgaaatcg tatcttcaga tgttgtaggt atgacattcg 840

gttcattatt tgatgcaact caaactcgtg taatgactgt ttcaggccgt caattagtta 900

aagtagcagc ttggtatgat aacgaaatgt c 931

<210> 6
<211> 931
<212> DNA
<213> Staphylococcus chromogenes

<400> 6
atggttttgg tagaattggt cgtttagcat tcagaagaat tcaagacgta gaaaatattg 60

aggttgtagc tgtaaacgat ttaacagacg acgatatgct tgcacattta ttgaaatatg 120

acacaatgca aggtcgtttt actgaagaag tagatgtaat tgatggtggt ttccgcgtaa 180

atggtaaaga agtgaaatca ttctctgaac cagaaccatc aaaattacca tggaaagatc 240

ttgacgtaga tgttgtttta gaatgtacag gtttctttac atcaaaagaa aaagcagaag 300

ctcacattga agcaggtgct aaaaaagtat taatttctgc accaggaact ggcgatctta 360

aaacaatcgt atataatgtc aaccatgaag aattagacgg ttctgaaaca gttgtatcag 420

gtgcttcttg tacaacaaac tcattagcac cagtagcaaa aactttaaat gatgaatttg 480

gtatcgttga aggtttaatg actacaattc acgcatacac tggtgaccaa aatacacaag 540

actcaccaca cagaaaaggt gacaaacgtc gtgcacgtgc agctgcagaa aacattattc 600

ctaactcaac aggtgctgcg aaagcaatcg gtttagttat ccctgaaatt gatggaaaat 660

tagacggtgg cgcacaacgt gtaccagtag caacaggttc attaactgaa ttaacagttg 720

ttttagataa agaagtatca gtagaagacg ttaacaatgc aatgaaaaat gcaacaaacg 780

aatcattcgg ttacactgaa gacgaaattg tatcttcaga tgttgtaggc atgactttcg 840

gagcattatt tgatgcaact caaacacgtg taatgactgt tggcgaccgt caattagtta 900

aagtagcagc ttggtatgat aacgaaatgt c 931

<210> 7
<211> 931
<212> DNA
<213> Staphylococcus cohni

<400> 7
atggttttgg tagaattggt cgtttagcat ttagaagaat tcaagatgta gaaggaatcg 60

atgtagtagc agtaaacgac ttaacagatg atgaaatgtt agctcattta ttaaaatatg 120

acactacaca aggtcgcttc acaggagaag ttgaagttga agaaaatggt ttccgcgtta 180

acggattaga agttaaatca ttttctgagc cagatccaag taaattacct tggggagact 240

tagatatcga tgttgtatta gaatgtactg gtttattcac agataaagaa aaagcagaag 300

cacacatcaa tgcaggcgct aaaaaagtat taatttcagc accagctaaa ggtgacttaa 360

aaacaatcgt atacaacact aaccacgaca ctttagatgg ttcagaagat gttgtttcag 420

gtgcttcatg tactactaac tcattagcac cagttgctaa agttttaaat ggtgaattcg 480

gcttaatcga aggtttcatg actacaatcc acgcatatac tggtgaccaa agcacacaag 540

atgcgcctca cagaaaaggt gacaaacgtc gtgcgcgtgc agctgctgaa aacattatcc 600

ctaactcaac aggtgctgct aaagctatcg gcctagtaat tcctgaaatt gatggtaaat 660

tagacggtgg agcacaacgt gtaccagttg caactggttc attaactgaa ttaacagtag 720

tattagataa aaatgtaagt atcgaagacg taaataacgc aatgaaaaat gcatctaacg 780

aatcattcgg ttatactgaa gacgaaatcg tttcttctga cgtaatcgga atgacatacg 840

gttcattatt tgatgcaact caaacacgtg ttatgactgt tggagatcgt caattagtta 900

aagtagcagc ttggtatgat aacgaaatgt c 931

<210> 8
<211> 931
<212> DNA
<213> Staphylococcus delphini

<400> 8
atggttttgg tagaattggt cgtttagcat ttagaagaat tcaagatgtg gaaaatatcg 60

acgttgtagc agtaaacgat ttaacagacg acgatatgct tgcacactta ttaaaatatg 120

actcaacaca aggtcgtttt actgaagaag tagaagtaat tgacggtggt ttccgtgtaa 180

atggtaaaga agtgaaatca ttctctgaac cagaacctaa aaacttacca tggggcgagt 240

tagacatcga cgtggtatta gaatgtactg gtttcttcac tgataaagaa aaagctgaag 300

cacacatcga agcaggcgcg aaaaaagtat taatttctgc accagctaaa ggtgacctta 360

aaacagttgt atataacgtt aaccacgaaa ttttagatgg tactgaaaca gttgtttctg 420

gtgcttcatg tacaacaaac tcattagcac ctgttgcaaa aactttacaa gacaacttcg 480

gtatcgttga aggtttaatg acaacaattc acgcttacac tggtgaccaa aacacattag 540

acgcacctca cagaaaaggt gacaaacgtc gtgcgcgtgc agctgctgaa aacatcatcc 600

ctaactcaac tggtgctgcg aaagcaatcg gtttagttat tcctgaaatc gatggtaaat 660

tagacggtgg tgcacaacgt gttcctgtag caacaggttc attaactgaa ttaactgtag 720

tattagaaaa agaagtctct gttgaagaag ttaacaaagt catgaaagag gcaactaacg 780

aatcattcgg ttacactgaa gacgaaatcg tttcttcaga cgttgttggt atgactttcg 840

gtgctttatt cgacgcaact caaacacgtg taatgactgt tggcgaccgt caattagtta 900

aagttgcagc ttggtatgat aacgaaatgt c 931

<210> 9
<211> 931
<212> DNA
<213> Staphylococcus epidermidis

<400> 9
atggttttgg tagaattggt cgtttagcat ttagaagaat tcaagacgta gaaggtttag 60

aagtggttgc agtaaacgac ttaactgatg acgatatgtt agcacactta ttaaaatatg 120

acactatgca aggtcgcttc actggtgaag tagaagttat cgacggtgga ttccgtgtta 180

acggtaaaga agttaaatca ttcgatgaac cagatgcaag caaattacct tggaaagact 240

tagatatcga cgtagtatta gaatgtactg gtttctatac tgacaaagat aaagcacaag 300

ctcatgttga cgcaggtgct aaaaaagtat taatctcagc tccagcaact ggtgacttaa 360

aaacaatcgt ttacaacact aaccacgatg aattagatgg ttctgaaaca gtagtatcag 420

gtgcatcatg tactactaac tcattagctc ctgtagctaa agtgttaagt gatgagttcg 480

gtttagttga aggtttaatg acaactatcc acgcttacac tggtgaccaa aatactcaag 540

atgctccaca cagaagaggc gacaaacgtc gtgctcgtgc tgcagcagaa aacatcatcc 600

ctaactcaac tggtgctgct aaagcaatcg gtaaagtaat tcctgaaatc gacggaaaat 660

tagacggtgg tgcacaacgt gttccagttg caactggttc attaactgaa ttaacagttg 720

tattagaaaa agacgtaaca gttgaacaag ttaacgaagc aatgaaacaa gcttctgacg 780

aatcattcgg ttacactgaa gacgaaatcg tatcttctga tgtagttggt atgacttacg 840

gttcattatt cgatgcaact caaactcgtg taatggctgt tggtggtcgt caattagtta 900

aagtagcagc ttggtatgat aacgaaatgt c 931

<210> 10
<211> 931
<212> DNA
<213> Staphylococcus equorum

<400> 10
atggttttgg tagaattggt cgtttagcat ttagaagaat tcaagacgta gaaggtattg 60

acgtagtagc agttaacgat ttaacagatg acgaaatgtt agctcattta ttaaaatatg 120

acactacaca aggtcgcttc acaggagaag ttgaagtaga aaaagacgga ttccgtgtaa 180

atggacaaga agttaaatca ttctcagaac ctgaaccaag taaattacct tggaaagatt 240

tagacatcga tgttgtttta gaatgtactg gtttcttcgc tgataaagaa aaagcagaag 300

ctcacattga cgctggcgct aaaaaagtat taatctctgc accagcaaca ggcgacttaa 360

aaacaatcgt ttataacact aaccacagtg aattagatgg ttcagaaaca gttgtttcag 420

gtgcttcatg tactactaac tcattagctc cagtagctaa agttttaaat gacgacttcg 480

gcttagttga aggtttcatg actactattc acgcatatac tggtgaccaa agtactcaag 540

atgctccaca cagaaaaggc gacaaacgcc gtgcacgtgc agctgctgaa aacatcatcc 600

ctaactcaac aggtgctgct aaagcaattg gtttagtaat ccctgaaatc gatggtaaat 660

tagacggtgg cgctcaacgt gttccagttg ctactggttc attaactgaa cttacagttg 720

tattagaaaa agacgtaagc gttgaagacg ttaacgcagc aatgaaaaat gcttcagacg 780

aatcatttgg ttacactgaa gacgaaatcg tttcttctga cgtaatcggt atgacttacg 840

gttcattatt cgatgcaacg caaactcgtg ttatgacagt tggagatcac caattagtta 900

aaatagcagc ttggtatgat aacgaaatgt c 931

<210> 11
<211> 931
<212> DNA
<213> Staphylococcus gallinarum

<400> 11
atggttttgg tagaattggt cgtttagcat tcagaagaat tcaaaacgtt gaaggaatcg 60

acgtagtagc agtaaatgac ttaacagatg acgaaatgtt agctcactta ttaaaatatg 120

atactatgca aggtcgcttc actggagaag ttgaagttga aaaagacggt ttccgtgtta 180

acggtcaaga agttaaatca ttctctgagc cagacccaag taaattacca tggggtgact 240

tagacatcga tgtagtatta gaatgtactg gtttcttcgc tgacaaaact aaagcagaag 300

ctcacatcaa tgcaggtgct aaaaaagtat taatctcagc tccagcaact ggtgacttga 360

aaacaatcgt tttcaacact aaccataacg aattagatgg tacagaaaca gttgtttcag 420

gtgcttcatg tactactaac tcattagctc cagtagctaa agtattaaat gatgactttg 480

gtttagttga aggtcttatg actacaattc acgcttacac tggtgaccaa aatacacaag 540

atgctccaca tgctaaaggt gacaaacgcc gtgctcgtgc agctgctgaa aatatcatcc 600

ctaactcaac tggtgctgct aaagcaatcg gtaaagttat ccctgaaatt gacggcaaat 660

tagacggtgg tgcgcaacgt gtaccagttg ctactggttc tttaactgaa ttaacagttg 720

tattagaaaa agacgtaagc gttgaagacg ttaacaatgc aatgaaaaac gcttcaaacg 780

aatcattcgg ttacactgaa gacgaaatcg tatcttctga cgtagttggt atcacttacg 840

gtccattatt cgatgcaaca caaactcgtg taatgactgt tggcgatcgt caattagtta 900

aagttgcagc ttggtatgat aacgaaatgt c 931

<210> 12
<211> 931
<212> DNA
<213> Staphylococcus haemolyticus

<400> 12
atggttttgg tagaattggt cgtttagcat ttagaagaat tcaagacgta ggaggtattg 60

aagtagttgc agtaaacgac ttaacagacg acgaaatgtt agctcattta ttaaaatatg 120

acactatgca aggtcgtttc acaggagaag ttgaagttgt tgatggtggt ttccgcgtaa 180

atggtaaaga agttaaatca tacgaagaac cagatgcaag caaattacct tggggcgatt 240

tagatatcga cgtagtatta gaatgtactg gtttctatac agataaagaa aaagcagaag 300

cacacatcaa tgcaggtgct aaaaaagtat taatctctgc accagctcaa ggtgatgtaa 360

aaacaatcgt attcaacact aaccacaatg acttagatgg ttcagaaaca gttgtttcag 420

gtgcatcatg tactactaac tcattagcac cagttgctaa agtattaagt gatgaatttg 480

gtttagttga aggtttaatg acaactattc acgcatacac tggtgaccaa atgactcaag 540

acggtccaca taaaaaaggt gacaaacgtc gtgcacgtgc agcagctcaa aacatcgtac 600

ctaactcaac aggtgctgca aaagcaatcg gtaaagttat tcctgaaatc gatggtaaat 660

tagacggtgg tgctcaacgt gtaccagttg ctacaggttc attaactgaa gtgacagttg 720

tattagaaaa agacgttact gttgaagacg ttaacaaagc aatgaaaaac gcttcaaacg 780

aatcatttgg ttacactgaa gacgaaatcg tttcttctga cgtagttggc atgacttacg 840

gttcattatt cgatgctact caaactcgtg taatgtctgt tggtgaccgt caattagtta 900

aagttgcagc ttggtatgat aacgaaatgt c 931

<210> 13
<211> 931
<212> DNA
<213> Staphylococcus hominis

<400> 13
atggttttgg tagaattggt cgtttagcat tcagaagaat tcaagacgta gaaggtattg 60

aagtagttgc agtaaacgac ttaacagacg acgaaatgtt agctcattta ttaaaatatg 120

acactatgca aggtcgcttt aacggagacg tagaagtagt tgaaggtggt ttccgtgtaa 180

atggtaaaga agttaaatct tttgaagaac cagatgcaag taaattacca tggggcgatt 240

tagatatcga cgtagtatta gaatgtactg gtttctatac agaaaaagaa aaagctgaag 300

cacacattaa tgcaggagct aaaaaagtat taat[?][?][?]tgc tccagccaaa ggtgatgtta 360

aaactatcgt atttaacaca aaccacaaag acttagatgg atctgaaaca gtagtatcag 420

gtgcttcatg tactacaaac tcattagcac cagttgctaa agttttaaat gacgaatttg 480

gtattgttga aggtttaatg acaactatcc atgcttacac tggtgaccaa atgactcaag 540

atggtccaca cagaaaaggt gacaaacgtc gtgcacgtgc agcagcacaa aacatcgtac 600

ctaactcaac aggtgcagct aaagctatcg gtaaagtaat tcctgaaatc gatggtaaat 660

tagatggtgg agcacaacgt gtaccagtag ctactggttc attaactgaa gtaacagttg 720

tattagaaaa agaagtaaca gttgaagatg ttaacaaagc aatgaaaaat gctgctgacg 780

aatcattcgg ttacactgaa gatgaaatcg tatcatcaga tgttgctggt atgaactttg 840

gttcattatt cgatgcaact caaactcgtg tcatctcagt tggcgacaaa caattagtta 900

aagtagcagc ttggtatgat aacgaaatgt c 931

<210> 14
<211> 931
<212> DNA
<213> Staphylococcus hyricus

<400> 14
atggttttgg tagaattggt cgtttagcat ttagaagaat tcaagacgta gaaaacattg 60

aggtagtagc tgtcaatgat ttaactgacg acgacatgct tgcacattta ttaaaatatg 120

acacgatgca aggacgtttt actgaagaag tagatgtaat tgatggtggt ttccgcgtaa 180

atggtaaaga agtgaaatca ttctctgaac cagaaccatc taaattacct tggaaagact 240

tagaagtaga tgttgtttta gaatgtactg gtttcttcac atctaaagaa aaagctgaag 300

cacacattga agcaggcgct aaaaaagtct taatttcagc accaggtact ggtgatctta 360

aaacaattgt atataacgtt aaccatgaag aattagacgg ttcagaaaca gttgtttcag 420

gtgcgtcttg tactacaaac tcattagctc cggtagcgaa aacattacac gatgaatttg 480

gtattgttga aggtttaatg actacaattc acgcttatac aggtgaccaa aatacgcaag 540

actcacctca cagaaaaggt gacaaacgtc gtgcacgtgc agctgctgaa aacatcatcc 600

ctaactcaac aggtgctgca aaagcaatcg gtttagttat tccagaaatt gctggtaaat 660

tagatggtgg cgcgcaacgt gtaccagttg ctacaggttc attaacagaa ttaactgttg 720

ttttagaaaa agaagtatct gttgaagaag ttaacaatgc aatgaaaaat gcaactaatg 780

aatcattcgg ttacactgaa gatgaaatcg tctcttctga cgttgtaggt atgacgtttg 840

gtgcattatt cgacgcaact caaacacgcg ttatgactgt tggcgatcgt caattagtta 900

aagttgcagc ttggtatgat aacgaaatgt c 931

<210> 15
<211> 934
<212> DNA

<213>  Staphylococcus intermedius

<400>  15
atggttttgg tagaattggt cgtttagcat tccgtcgtat tcaaaatgtg gaaggaattg      60

aagttgttgc aatcaatgac ttaacagacg ctaaaatgtt agctcatctg ttaaaatatg     120

atacaactca aggccgtttt gacggtgaag tagaagtaca tgatggtttc ttcaaagtaa     180

acggtaaaga agttaaagta ttagctaacc gtaacccaga agaacttcca tggggtgaac     240

taggagtaga catcgttctt gaatgtactg gtttcttcac agcacaagac aaagctgaat     300

tacacattaa agctggcgct aaaaaagttg ttatctccgc tccagcaact ggcgacatga     360

aaacaatcgt ttacaatgta aaccatgaaa cattagacgg aactgaaaca gttatttctg     420

gtgcaagctg tactactaac tgtttagctc caatggctaa agttttagaa gacaaatttg     480

gtgttgttga aggcctaatg actacaattc acgcatacac tggtgaccaa aatacattag     540

acgctccaca tccaaaaggt gacttccgtc gtgctcgtgc tgctgcagaa aatatcatcc     600

ctaatacaac tggtgctgca aaagctatcg gtgaagtatt accaagcctt aaaggtaaat     660

tagacggagc agctcaacgt gttccagttc caactggttc ccttactgaa ttagtaacag     720

ttcttaacaa aaaagttact gttgatgaag taaatgcagc tatggaagca gcttctgatc     780

cagaaacatt cggttacact aatgacgcaa tcgtttcttc tgatatcaaa ggtatgactt     840

tcggttcttt atttgacgaa actcaaacaa aagttcttac agttggcgat caacaattag     900

ttaaaactgc agcttggtat gataacgaaa tgtc                                 934


<210>  16
<211>  932
<212>  DNA
<213>  Staphylococcus kloosii

<400>  16
atggttttgg tagaattggt cgtttagcat ttagaagaat tcaaaacgtt gacggaatcg      60

atgtagtagc agttaacgac ttaacagatg acgaaatgtt agcacactta ttaaaatatg     120

acacaatgca aggtcgtttc actggagaag ttgaagttga agaaaacggc ttccgcgtaa     180

atggtcaaga agttaaatca ttctctgaac cagatccaag taaattacca tggggcgact     240

tagatatcga tgttgtctta gaatgtactg gtttatttgc tgataaagat aaagcttcag     300

ctcatatcga tgcaggcgct aaaaaagttt taatttcagc tccagctaca ggcgacttaa     360

aaacaatcgt ttacaacact aaccacaacg aattagacgg ttcagaatca gtagtatcag     420

gtgcttcatg tactactaac tcattagctc cagtagctaa agttttaaat gatgaattta     480

gtttagttga aggtttaatg acaactatcc acgcttacac tggtgaccaa agcacacaag     540

atgctcctca cagaaaaggt gacaaacgtc gtgctcgtgc agcagcagaa aacatcatcc     600

ctaactcaac aggtgctgca aaagcaatcg gtttagttat tcctgaaatc gacggaaaat     660

tagacggtgg cgcacaacgt gttccagttg caacaggttc attaactgaa ttaacagttg 720

tattagaaaa agacgtaagt gttgaagatg taaacaacgc aatgaaaaat gcttcaaacg 780

aatcatttgg ttacactgaa gacgaaatcg tttcttctga cgtaatcggt atgacttacg 840

gttcattatt cgacgctaca caaactcgtg taatgactgt tggtgactcg tcaattagtt 900

aaagttgcag cttggtatga taacgaaatg tc 932

<210> 17
<211> 931
<212> DNA
<213> Staphylococcus lentus

<400> 17
atggttttgg tagaattggt cgtttagcat ttagaagatt acaagaagta gaaaatatcg 60

aagtagtagc aatcaacgat ttagcagatg acgctatgtt agctcattta ttaaaatacg 120

attctacaca aggtcgtttc aaagatgaag tagaagtaat tgaaggcgga ttccgtgtaa 180

acggtaaaga aatcaaaact ttcgaaaatc ctaaccctaa agaattaccc tggggagact 240

tagacatcga tgtagtatta gaatgtactg gtttcttcgc tgataaagaa aaagctcatg 300

ctcacatcgc cgcaggtgct aaaaaagtat taatttcagc tccagcttca ggcgacttga 360

aaacaatcgt atacaatgtt aaccatgatg aattagacgg ttcagaagaa atcgtatctg 420

gtgcatcttg tactactaac tgtttagctc caatggctaa agtattaaat gatgaattcg 480

gtatcgttga aggattaatg atgacaattc atgcttatac tggtgaccaa aatacactag 540

atgctccaca tgctaaaggt ggcttccgtc gtgctcgtgc agcagctgaa aacatcgtac 600

ctaactcaac tggtgcagct aaagcaattg gcttagttat cccagaatta aaaggtaaat 660

tagatggatc agctcaacgt gttccagtag caactggttc agtaactgaa ttaacagcag 720

tattagataa agaagtatct atagaagaaa tcaacgaagc aatgaaaaat gctacaaatg 780

attcattcgg atacactgaa gacgaaatcg tttcttctga tgttattggc atcacttacg 840

gttcattatt cgacgcaact caaactcgtg taatgacagt tggagaccgt caattagtta 900

aaactgcagc ttggtatgat aacgaaatgt c 931

<210> 18
<211> 934
<212> DNA
<213> Staphylococcus lugdunesis

<400> 18
atggttttgg tagaattggt cgtttagcat ttagaagaat tcaagacgta gaaggcatcg 60

aggtagtagc agtaaacgac ttaacagatg atgatatgtt agcgcattta ttaaaatatg 120

acactatgca aggtcgcttc actagcgaag ttgaagttgt tgatggtggt ttccgtgtaa 180

atggtaaaga agttaaatca tttgaagaac cagacgcaag caaattacca tggggtgacc 240

taggtgttga cgtggtatta gaatgtactg gattctatac agataaagaa aaagctgaag 300

cacacattca tgcaggtgct aaaaaagtat taatttctgc gccagctaaa ggtgacgtta 360

aaactatcgt ttacaacact aaccatagtg acttagacgg ttcagaaaca gttgtatcag 420

gtgcttcatg tacaactaac tctttagcac cagtagctaa agtaatcagc gatgaatttg 480

gtttagtaga aggtttaatg acaactattc atgcatacac tggtgaccaa atgactcaag 540

atggtccaca cagaaaaggt gacaaacgtc gtgctcgtgc agctgcacaa aacatcgtac 600

ctaactcaac tggtgctgct aaagcaatcg gtaaagttat tcctgaaatc gatggtaaat 660

tagacggtgg tgcgcaacgt gtacctgtag ctacaggttc attaactgaa ttaactgtag 720

tattagaaaa acaagatgta acagtagaac aagtaaacga agcgatgaaa aaagcttcta 780

acgaatcatt cggttacaat gaagatgaaa ttgtttcttc tgacgtagtt ggtatgactt 840

acggttcatt atttgatgca acacaaactc gtgtaatgtc agttggtggc cgtcaattag 900

ttaaagttgc agcttggtat gataacgaaa tgtc 934

<210> 19
<211> 931
<212> DNA
<213> Staphylococcus piscifermentans

<400> 19
atggttttgg tagaattggt cgtttagcat tcagaagaat tcaagatgtt gaaggtatcg 60

atgtagtagc agtaaacgac ttaacagatg atgaaatgtt agcgcattta ttaaaatacg 120

atacaatgca aggacgtttc acagaagaag ttgaagttgt agatggcgga ttccgtgtga 180

atggtaaaga agttaaatca ttcgaagaac cagatgcaag caaattacct tggaaagatt 240

tagacattga tgttgtatta gaatgtactg gtttctatac aagtgatgaa aaagctaaag 300

cacatatcga cgcaggtgct aaaaaagtat taatttctgc tccagcaact ggcgatgtta 360

aaacaatcgt ttataacgta aaccaagata ctttagacag ctctgatgtt atcgtttcag 420

gtgcttcttg tactacaaac tcacttgctc cagtagcaaa agttttaaac gacagctttg 480

gtttagttga aggtttcatg actactattc atgcttacac tggtgaccaa aatactcaag 540

atggtccaca cagaaaaggt gacaaacgtc gtgcacgtgc agcagctcaa aacatcgtac 600

caaactcaac tggtgctgct aaagcaatcg gtaaagtaat ccctgaaatt gacggtaaat 660

tagacggtgg tgctcaacgt gttcctgttg caactggttc attaacagaa ttaacagttg 720

tattagacaa agaagtttca gttgacgaag taaacgaagc aatgaaacaa gcttctaacg 780

aatcattcgg ttacaatgaa gacgaaatcg tatcttctga cgtggttggt atgacattcg 840

gttcattatt c[?]atgctaca caaactcgtg tgatgactgt atcaggtcgt caattagtta 900

aagttgcagc ttggtatgat aacgaaatgt c 931

<210> 20
<211> 931
<212> DNA
<213> Staphylococcus saprophyticus

<220>
<221> misc_feature
<222> (727)..(733)
<223> n = a or g or c or t/u, unknown or other

<400> 20
atggttttgg tagaattggt cgtttagcat tcagaagaat tcaaaacgtt gacggaatcg 60

acgtagtagc agtaaacgat ttaacagatg acgaaatgtt agctcattta ttaaaatatg 120

atactatgca aggacgcttc acaggagaag ttgaagtaga aaaagacggt ttccgcgtaa 180

acggacaaga agtaaaatca ttctctgagc ctgaaccaag taaattacct tggaaagact 240

tagacatcga tgttgtatta gaatgtactg gtttcttcgc tgataaagaa aaagcagaag 300

cacacatcaa tgcaggtgct aaaaaagtat taatctctgc tccagctaca ggcgatttaa 360

aaacaatcgt ttataataca aaccaccaag aattagacgg ttcagaaact gttgtttcag 420

gtgcttcatg tactactaac tcattagctc ctgttgctaa agttttaaat gatgacttcg 480

gtttagtaga aggtttcatg actactatcc acgcatacac tggtgaccaa agcacactag 540

atgcaccaca cagaaaaggc gacaaacgtc gtgcgcgtgc agctgctgaa aacatcatcc 600

ctaactcaac tggtgctgct aaagcaattg gcttagtaat tcctgaaatt gatggtaaat 660

tagatggagg agcgcaacgt gttcctgttg caactggttc attaactgaa ttaacagttg 720

ttttagnnnn nnntgtaagc attgaagatg taaatgctgc aatgaaaaat gcttcaaacg 780

aatcattcgg ttacacagaa gacgaaatcg tatcttcaga cgtaatcggt atgacttatg 840

gttcattatt tgatgcaaca caaactcgtg taatgactgt tggcgaccgt caattagtta 900

aagtagcagc ttggtatgat aacgaaatgt c 931

<210> 21
<211> 931
<212> DNA
<213> Staphylococcus schleiferi

<400> 21
atggttttgg tagaattggt cgtttagcat ttagaagaat tcaagatgta gaaaacattg 60

aggtcgtagc tgtaaacgat ttaacagatg acgatatgct tgcacattta ttgaaatatg 120

acacaatgca aggacgtttt actgaagaag tggaagtaat tgatggtggt ttccgtgtga 180

atggtaaaga agcgaaatca ttctctgaac cagaacctgc taaattacct tggggtgacc 240

ttggtgtgga cgtagtatta gaatgtactg gtttcttcac agataaagaa aaagctgaag 300

cacacattca agcaggcgct aaaaaagtat taatctcagc accggctaaa ggtgatctta 360

aaacaatcgt atataatgtt aaccacgacg atttagatgg ttctgaaaca gttgtttcag 420

gtgcatcatg tactacaaac tcattagcac ctgttgcaaa aactttacac gacgaattcg 480

gtattgttga aggtttaatg actacaattc acgcatatac tggtgaccaa aatacacaag 540

atgcacctca cagaaaaggt gacaaacgtc gtgcgcgtgc tgctgctgaa aacattatcc 600

ctaactctac aggggcagca aaagcaatcg gtttagttat tccagaaatt gccggtaaat 660

tagacggtgg tgcacaacgt gttccagttg caactggttc attaacagaa ttaacagttg 720

ttttagataa agaagtgact gttgaagaag taaacaaagt attgaaagca gcaactaacg 780

aaccattcgg ttacactgaa gacgaaattg tttcttcaga cgttgtaggt atgacttacg 840

gtgcattatt cgatgcaact caaactcgtg taatgactgt tggcgaccgt caattagtta 900

aagttgcagc ttggtatgat aacgaaatgt c 931

<210> 22
<211> 931
<212> DNA
<213> Staphylococcus sciuri

<400> 22
atggttttgg tagaattggt cgtttagcat ttagaagatt acaagaagtt gaaaatatcg 60

aagtagtagc aatcaacgat ttaacagatg acgcaatgtt agctcattta ttaaaatatg 120

attcaacaca aggtcgtttc aaagacgaag tagaagttat cgaaggcgga ttccgcgtaa 180

acggtagaga aatcaaaact ttcgaaaatc ctaatcctaa agaattacca tggggcgatt 240

tagatatcga tgtagtatta gaatgtactg gtttcttcgc tgacaaagac aaagcttcag 300

ctcacatcga agcaggtgct aaaaaagtat taatttcagc tccagcatca ggtgacttaa 360

aaactatcgt ttataacgtt aaccatgacg aattagacgg atctgaagaa atcgtttcag 420

gtgcatcttg tacaactaac tgtttagctc caatggctaa agtattaaat gatgaattcg 480

gtatcgttga aggtttaatg atgacaattc acgcttacac tggtgaccaa aatactttag 540

atgctccaca tgctaaaggt gacttccgtc gtgctcgtgc agctgctcaa aacatcgtgc 600

ctaactcaac tggtgctgct aaagcaatcg gtttagtaat tccagaatta aaaggtaaat 660

tagatggatc agctcaacgt gttccagtag caactggttc agtaacagaa ttaacagctg 720

tattggacaa agaagtttca gttgaagaaa tcaatgcagc aatgaaaaat gctacaaatg 780

attcattcgg ttacactgaa gacgaaatcg tatcttctga tatcattggt atcacttacg 840

gttcattatt tgatgcaact caaactcgtg ttatgacagt tggagatcgc caattagtta 900

aaactgcagc ttggtatgat aacgaaatgt c 931

<210> 23
<211> 932
<212> DNA
<213> Staphylococcus simulans

<400> 23
   atggttttgg tagaattggt cgtttagcat ttcagaagaa ttcaagatgt tgaaggtatc 60
   gatgtagtag cagtaaacga cttaacagat gatgaaatgt tagcacactt attaaaatac 120
   gatacaatgc aaggacgttt cactgaagaa gttgaagttg tagatggcgg attccgcgtg 180
   aatggtaaag aagttaaatc attcgaagaa ccagatgcaa gcaaattacc ttggaaagat 240
   ttagacattg atgtcgtatt agaatgtact ggtttctaca ctagcgacga aaaagcacaa     300
   gctcacattg acgcaggtgc taaaaaagta ttaatctctg caccagcaac tggtgacgtt     360
   aaaacaatcg tttataacgt aaaccaagat actttagaca gctctgacgt aatcgtttca 420
   ggtgcttctt gtactacaaa ctcacttgct ccagtagcaa aagtattaaa tgacagcttc 480
   ggtttagtag aaggtttcat gactactatc cacgcttaca ctggtgacca aaatactcaa 540
   gacggtccac acagaaaagg cgacaaacgt cgtgcacgtg cagcagctga aaacatcgtt 600
   cctaactcaa ctggtgctgc taaagcaatc ggtaaagtaa ttcctgaaat cgacggaaaa 660
   ttagacggtg gcgctcaacg tgttcctgta gcaactggtt cattaactga attaacagtt 720
   gtattagaca aagacgtaac aatcgaagaa gtaaacgaag ctatggaagc agcttctaac 780
   gaatcattcg gttacaacga agacgaaatc gtatcttcag acgtagttgg tatgacattc 840
   ggttcattat tcgatgcaac tcaaactcgt gttatgactg tatctggtcg tcaattagtt 900
   aaagttgcag cttggtatga taacgaaatg tc 932

<210> 24
<211> 931
<212> DNA
<213> Staphylococcus xylosus

<400> 24
atggttttgg tagaattggt cgtttagcat ttagaagaat tcaaaacgtt gacggaattg 60

acgtagtagc agtaaatgac ttaacagatg acgaaatgtt agcacattta ttaaaatatg 120

acactatgca aggacgcttc acaggagaag ttgaagtaga aaatgacggt ttccgtgtaa 180

acggacaaga agtaaaatca ttctctgagc cagacccaag caaattacct tggaaagatt 240

tagacatcga tgttgtatta gaatgtactg gtttctacgc tgataaagaa aaagcagaag 300

ctcacattaa tgcaggtgct aaaaaagtat taatttcagc tccagctact ggtgatttaa 360

aaacaatcgt ttataacaca aaccaccaag agttagatgg taaagaaaca gttgtttcag 420

gtgcttcatg tacaactaac tcattagctc cagttgctaa agtattaaat gatgactttg 480

gtttagtaga aggtttcatg actacaattc atgcttacac tggtgaccaa aatacacaag 540

atgcgccaca cgctaaaggc gacaaacgtc gtgctcgtgc agctgctgaa aacattatcc 600

ctaactcaac tggtgctgct aaagcaattg gcctagttat ccctgaaatt gatggtaaat 660

tagacggtgg agcgcaacgt gttcctgtag ctactggttc gttaacagaa ttaacagttg 720

tattagacaa aaatgtaagt gttgaagacg ttaatgctgc aatgaagaat gcttcaaacg 780

aatcattcgg ttacactgaa gacgaaatcg tttcttctga tgtagttggt atgacttacg 840

gttcattatt cgatgcaaca caaacacgtg ttatgacagt tggcgatcgc caattagtta 900

aagtagcagc ttggtatgat aacgaaatgt c 931

<210> 25
<211> 747
<212> DNA
<213> Staphylococcus caseolyticus

<220>
<221> misc_feature
<222> (683)..(683)
<223> n = a or g or c or t/u, unknown or other

<400> 25
atggttttgg tagaattggt cgtttattag aacagcatgc acgggtaaaa gcattggatg 60

cacaatatcc agaaattgat atttattcag gtgttgagat ggatatatta gcagatggcg 120

aaatggatta tagcaatgac gtgctagcac agcttgacta ttgtattggt gctattcatc 180

agtcgttgaa tcaaagtgaa gatgagatta tgaaacgcct gatcaatgcc tgtaataatc 240

catacattag acatattgct catccaacgg gaaggttgat tggtcgccgt aatggttatc 300

atgtaaacat gccgaaactc atcgaaactg cacagaagac aaataccatc cttgaaatca 360

atgcacatcc gatgcgcctt gatttatcga gcgacgtatt aaagcaatat ccagatatta 420

aacttgtgat caacacagac gcgcgtgcaa tcgatcagct tgatttaatg aaatatggtg 480

tgggtacagc aataaaaggc catgtgaaaa aagaacaggt aataaacact ttaccgcgta 540

aggattttaa atcttggata cagaatggga agtaattata tgaataaaaa agcattaaat 600

atattagaat ataacaaaat tattgagcgt gttgacgcat ttactcaaaa tgaactttca 660

agcaaaaagt gaggatgaca cancctatca gcgacaaagc tgaaatagat agcatgcttg 720

cacagcttgg tatgataacg aaatgtc 747

<210> 26
<211> 934
<212> DNA
<213> Staphylococcus aureus

<400> 26
atggttttgg tagaattggt cgtttagcat tcagaagaat tcaagaagta gaaggtcttg 60

aagttgtagc agtaaacgac ttaacagatg acgacatgtt agcgcattta ttaaaatatg 120

acactatgca aggtcgtttc acaggtgaag tagaggtagt tgatggtggt ttccgcgtaa 180

atggtaaaga agttaaatca ttcagtgaac cagatgcaag caaattacct tggaaagact 240

taaatatcga tgtagtgtta gaatgtactg gtttctacac tgataaagat aaagcacaag 300

ctcatattga agcaggcgct aaaaaagtat taatctcagc accagctact ggtgacttaa 360

aaacaatcgt attcaacact aaccaccaag agttagacgg ttctgaaaca gttgtttcag 420

gtgcttcatg tactacaaac tcattagcac cagttgctaa agttttaaac gatgactttg 480

gtttagttga aggtttaatg actacaattc acgcttatac aggtgatcaa aatacacaag 540

acgcacctca cagaaaaggt gacaaacgtc gtgctcgtgc agcagcagaa aacatcatcc 600

ctaactcaac aggtgctgct aaagctatcg gtaaagttat tcctgaaatc gatggtaaat 660

tagatggtgg tgcacaacgt gttcctgtag ctacaggttc attaactgaa ttaacagtag 720

tattagaaaa acaagacgta acagttgaac aagttaacga agctatgaaa aatgcttcaa 780

acgaatcatt cggttacact gaagacgaaa tcgtttcttc agacgttgta ggtatgactt 840

acggttcatt atttgacgct acacaaactc gtgtaatgtc agttggcgac cgtcaattag 900

ttaaagttgc agcttggtat gataacgaaa tgtc 934

<210> 27
<211> 931
<212> DNA
<213> Staphylococcus warneri

<400> 27
atggttttgg tagaattggt cgtttagcat ttagaagaat tcaagacgta gaaggtttag 60

aagtagttgc agtaaacgac ttaactgatg acgatatgtt agcacactta ttaaaatatg 120

acactatgca aggtcgcttc actggtgaag tagaagttat cgacggtgga ttccgtgtta 180

acggtaagga agttaaatca ttcgatgaac cagatgcaag caaattacct tggaaagact 240

tagatatcga cgtagtatta gaatgtactg gtttctatac tgacaaagat aaagcacaag 300

ctcatgatga cgcaggtgct aaaaaagtat taatctcagc tccagcaact ggtgacttaa 360

aaacaatcgt ttacaacact aaccacgatg aattagatgg ttctgaaaca gtagtatcag 420

gtgcatcatg tactactaac tcattagctc ctgtagctaa agtgttaagt gatgagttcg 480

gtttagttga aggtttaatg acaactatcc acgcttacac tggtgaccaa aatactcaag 540

atgctccaca cagaaaaggc gacaaacgtc gtgctcgtgc tgcagcagaa aacatcatcc 600

ctaactcaac tggtgctgct aaagcaatcg gtaaagtaat tcctgaaatc gacggaaaat 660

tagacggtgg tgcacaacgt gttccagttg caactggttc attaactgaa ttaacagtag 720

tattagaaaa agacgtaaca gttgaacaag ttaacgaagc aatgaaacaa gcttctgacg 780

aatcattcgg ttacactgaa gacgaaatcg tatcttctga tgtagttggt atgacttacg 840

gttcattatt cgatgcaact caaactcgtg taatggctgt tggtggtcgt caattagtta 900

aagtagcagc ttggtatgat aacgaaatgt c 931

<210> 28
<211> 880
<212> DNA
<213> Staphylococcus arlettae

<400> 28
gcatttagaa gaattcaaaa cgttgatgga atcgacgttg tagcagtaaa cgacttaaca 60

gatgatgaaa tgttagcaca cttattaaaa tatgacacta tgcaaggacg tttcacagga 120

gaagttgaag tagaaaatga cggtttccgt gtaaatggtc aagaagttaa atcattctca 180

gaaccagatc caaataaatt accatggggc gacttagata tcgatgttgt tttagaacgt 240

actggtttat tcgcagacaa agataaagca tcagctcata tcgacgcagg tgcgaaaaaa 300

gtattaatct ctgctccagc atcaggtgat ttaaaaacaa tcgtattcaa cactaaccac 360

aatgaattag atggttctga aacagttgta tcaggtgctt catgtactac taactcatta 420

gctccagttg ctaaagtatt aaacgatgac ttcggtttag tagaaggttt aatgactact 480

atccatgctt acactggtga ccaaagcaca caagacgctc ctcacagaaa aggtgacaaa 540

cgtcgtgcgc gtgcagctgc tgaaaacatt attcctaact caacaggtgc tgctaaagca 600

atcggtttag ttattcctga aatcgatggt aaattagatg gtggcgctca acgtgtacca 660

gtagctactg gttcattaac tgaattaaca gttgtattag acaaagaagt aactgttgaa 720

gacgttaata gtgcaatgaa aaatgcttca aacgaatcat ttggttacac tgaagacgaa 780

atcgtttctt ctgacgtaat cggtatgact tacggttcat tattcgatgc tacacaaact 840

cgtgtaatga ctgttggtaa ccaacaaatg gttaaagtag 880

<210> 29
<211> 880
<212> DNA
<213> Staphylococcus auricularia

<400> 29
gcattcagaa gaattcaaaa tgttgaaggc attgacgtag tagcagttaa tgacttaaca 60

gatgacgata tgttagcaca cttattaaaa tacgacacaa tgcaaggtcg tttcacaagt 120

gaagttgaaa tcgtagaagg cggtttccgc gttaacggtc aagaagtgaa atcattcgac 180

gaaccagatc caagcaaatt accttggaaa gacttagaca tcgacgttgt acttgaatgt 240

actggtttat tcacagacaa agaaaaagca gaagcacaca tcgacgcagg tgctaaaaaa 300

gtattaatct ctgcaccagc taaaggtgac gttaaaacag ttgtttataa cactaaccac 360

gatacacttg atggtacaga aacagttgtt tcaggtgctt catgtacaac aaactcatta 420

gcaccagttg ctaaagtact taacgatgaa ttcggtttag tagaaggatt catgactaca 480

atccacgcat acactggtga ccaaaataca caagatgcac cacacaaaaa aggtgacaaa 540

cgccgtgcac gtgcagctgc tcaaaacatc atccctaact ctacaggtgc tgcaaaagca 600

atcggtaaag ttatcccaga aatcgacggt aaattagacg gcggtgcaca acgtgtccct 660

gttgctactg gttcattaac agaattaaca gtagtattag aaaaagatgt ttcaatcgaa 720

gacgtaaaca acgcaatgaa aaatgcatca actgaatcat tcggttacac agatgacgaa 780

atcgtttctt ctgacgtcat cggtatgact tatggttcat tattcgacgc aactcaaact 840

cgtgtcatga ctgttggcga tcaccaatta atcaaagttg 880

<210> 30
<211> 883
<212> DNA
<213> Staphylococcus caprae

<400> 30
gcattcagaa gaatccaaga tgtagaaggt cttaaagtag tagcagttaa cgacttaaca 60

gacgatgaaa tgttagctca tttattaaaa tatgacacta tgcaaggacg cttcactgga 120

gaagttgaag ttatcgaagg cggattccgc gtaaacggta aagaaattaa atcattcgac 180

gaaccagatg caaacaaatt accttggggt gaccttgaca tcgatgtagt attagaatgt 240

actggtttct acactgataa agataaagca caagcacata tcgatgcagg tgctaaaaaa 300

gtattaatct cagctccagc tactggtgat ttaaaaacaa tcgtattcaa tactaaccat 360

gacgaattag atggttcaga aacagttgta tcaggtgctt cttgtacaac taactcatta 420

gctcctgttg ctaaagtatt aaatgatgaa ttcggtttag ttgaaggttt aatgacaact 480

atccacgctt acacaggtga ccaaaatact caagacgcac ctcacagaaa aggtgacaaa 540

cgtcgtgctc gtgcagcagc agaaaacatt atccctaact caacaggtgc tgctaaagca 600

atcggtaaag taattcctga aatcgacggt aaattagacg gtggtgcaca acgtgttcca 660

gttgctacag gttcattaac agaattaact gtagtattag aaaaacaaga tgtaactgct 720

gatcaagtta acgaagcaat gaaacaagct tctgacgaat cattcggtta cactgaagat 780

gaaatcgtat cttctgacgt tgtaggtatc acttatggtt cattattcga tgcaactcaa 840

actcgtgtaa tgactgttgg tgaccgtcaa ttagttaaag tag 883

<210> 31
<211> 883
<212> DNA
<213> Staphylococcus capitis

<400> 31
gcattcagaa gaatccaaga tgtagaaggt ctagaagtag ttgcagttaa cgacttaaca 60

gatgatgaaa tgttagctca cttattaaaa tatgacacta tgcaaggtcg cttcactgga 120

gaagttgaag tcatcgatgg tggattccgt gttaacggta aagaaattaa atcattcgat 180

gaaccagatg caagcaaatt accttgggga gacctagaca tcgacgtagt attagaatgt 240

actggtttct acactgacaa agataaagca caagctcaca tcgatgcagg tgctaaaaaa 300

gtattaatct ctgctccagc tacaggtgat ttaaaaacaa tcgtattcaa cactaaccat 360

gatgaattag atggttctga aacagttgta tcaggtgctt cttgtacaac taactcatta 420

gcaccagttg ctaaagtttt aaatgatgag tttggtttag ttgaaggttt aatgactact 480

atccacgctt atactggtga ccaaaatact caagatgctc ctcacagaaa aggcgacaaa 540

cgtcgtgctc gtgcagcagc agaaaacatt atccctaact ctacaggtgc tgctaaagca 600

attggtaaag ttattccaga aatcgatggt aaattagacg gtggagctca acgtgttcca 660

gttgcaacag gttcattaac tgaattaaca gtagtattag ataaacaaga tgtaacagct 720

gatcaagtaa atgaagcaat gaaacaagct tctgacgaat cattcggtta cactgaagat 780

gaaatcgtac cttctgacgt tgtaggtatg acttatggtt cattattcga cgctactcaa 840

actcgtgtaa tgactgttgg tgaccgtcaa ttagttaaag ttg 883

<210> 32
<211> 880
<212> DNA
<213> Staphylococcus carnosus

<400> 32
gcatttagaa gaattcaaga tgttgaaggt atcgatgtag tagcagtaaa cgacttaaca 60

gatgatgaaa tgttagcaca cttattaaaa tacgatacaa tgcaaggacg tttcactgaa 120

gaagttgaag ttgtagatgg cggattccgc gtgaatggta aagaagttaa atcattcgaa 180

gaaccagatg caagcaaatt accttggaaa gatttagaca ttgatgttgt attagaatgt 240

acaggtttct atacaagtga agaaaaagca aaagctcata tcgatgcagg cgctaaaaaa 300

gtattaattt cagcaccagc tactggtgat gttaaaacaa tcgtttataa cgtaaaccaa 360

gatactttag acagctctga cgtaatcgtt tcaggtgctt cttgtactac aaactctctt 420

gctcctgtag cacaagtttt aaatgacagc tttggtttag ttgaaggttt catgactact 480

atccatgctt atactggtga ccaaaatact caagatggtc cacacagaaa aggtgacaaa 540

cgtcgtgctc gtgcagcagc tgaaaacatc gttccaaact caactggtgc agctaaagca 600

attggtaaag taattcctga aatcgacgga aaattagaca gcggcgctca acgtgttcct 660

gttgcaactg gttcattagc tgaattaaca gttgtattag ataaagacgt tacagttgac 720

gaagtaaacg aagcaatgaa acaagcttct aacgaatcat tcggttacaa cgaagacgaa 780

atcgtatctt cagatgttgt aggtatgaca ttcggttcat tatttgatgc aactcaaact 840

cgtgtaatga ctgtttcagg ccgtcaatta gttaaagtag 880

<210> 33
<211> 880
<212> DNA
<213> Staphylococcus chromogenes

<400> 33
gcattcagaa gaattcaaga cgtagaaaat attgaggttg tagctgtaaa cgatttaaca 60

gacgacgata tgcttgcaca tttattgaaa tatgacacaa tgcaaggtcg ttttactgaa 120

gaagtagatg taattgatgg tggtttccgc gtaaatggta aagaagtgaa atcattctct 180

gaaccagaac catcaaaatt accatggaaa gatcttgacg tagatgttgt tttagaatgt 240

acaggtttct ttacatcaaa agaaaaagca gaagctcaca ttgaagcagg tgctaaaaaa 300

gtattaattt ctgcaccagg aactggcgat cttaaaacaa tcgtatataa tgtcaaccat 360

gaagaattag acggttctga aacagttgta tcaggtgctt cttgtacaac aaactcatta 420

gcaccagtag caaaaacttt aaatgatgaa tttggtatcg ttgaaggttt aatgactaca 480

attcacgcat acactggtga ccaaaataca caagactcac cacacagaaa aggtgacaaa 540

cgtcgtgcac gtgcagctgc agaaaacatt attcctaact caacaggtgc tgcgaaagca 600

atcggtttag ttatccctga aattgatgga aaattagacg gtggcgcaca acgtgtacca 660

gtagcaacag gttcattaac tgaattaaca gttgttttag ataaagaagt atcagtagaa 720

gacgttaaca atgcaatgaa aaatgcaaca aacgaatcat tcggttacac tgaagacgaa 780

attgtatctt cagatgttgt aggcatgact ttcggagcat tatttgatgc aactcaaaca 840

cgtgtaatga ctgttggcga ccgtcaatta gttaaagtag 880

<210> 34
<211> 880
<212> DNA
<213> Staphylococcus cohni

<400> 34
gcatttagaa gaattcaaga tgtagaagga atcgatgtag tagcagtaaa cgacttaaca 60

gatgatgaaa tgttagctca tttattaaaa tatgacacta cacaaggtcg cttcacagga 120

gaagttgaag ttgaagaaaa tggtttccgc gttaacggat tagaagttaa atcattttct 180

gagccagatc caagtaaatt accttgggga gacttagata tcgatgttgt attagaatgt 240

actggtttat tcacagataa agaaaaagca gaagcacaca tcaatgcagg cgctaaaaaa 300

gtattaattt cagcaccagc taaaggtgac ttaaaaacaa tcgtatacaa cactaaccac 360

gacactttag atggttcaga agatgttgtt tcaggtgctt catgtactac taactcatta 420

gcaccagttg ctaaagtttt aaatggtgaa ttcggcttaa tcgaaggttt catgactaca 480

atccacgcat atactggtga ccaaagcaca caagatgcgc ctcacagaaa aggtgacaaa 540

cgtcgtgcgc gtgcagctgc tgaaaacatt atccctaact caacaggtgc tgctaaagct 600

atcggcctag taattcctga aattgatggt aaattagacg gtggagcaca acgtgtacca 660

gttgcaactg gttcattaac tgaattaaca gtagtattag ataaaaatgt aagtatcgaa 720

gacgtaaata acgcaatgaa aaatgcatct aacgaatcat tcggttatac tgaagacgaa 780

atcgtttctt ctgacgtaat cggaatgaca tacggttcat tatttgatgc aactcaaaca 840

cgtgttatga ctgttggaga tcgtcaatta gttaaagtag 880

<210> 35
<211> 880
<212> DNA
<213> Staphylococcus delphini

<400> 35
gcatttagaa gaattcaaga tgtggaaaat atcgacgttg tagcagtaaa cgatttaaca 60

gacgacgata tgcttgcaca cttattaaaa tatgactcaa cacaaggtcg ttttactgaa 120

gaagtagaag taattgacgg tggtttccgt gtaaatggta aagaagtgaa atcattctct 180

gaaccagaac ctaaaaactt accatggggc gagttagaca tcgacgtggt attagaatgt 240

actggtttct tcactgataa agaaaaagct gaagcacaca tcgaagcagg cgcgaaaaaa 300

gtattaattt ctgcaccagc taaaggtgac cttaaaacag ttgtatataa cgttaaccac 360

gaaattttag atggtactga aacagttgtt tctggtgctt catgtacaac aaactcatta 420

gcacctgttg caaaaacttt acaagacaac ttcggtatcg ttgaaggttt aatgacaaca 480

attcacgctt acactggtga ccaaaacaca ttagacgcac ctcacagaaa aggtgacaaa 540

cgtcgtgcgc gtgcagctgc tgaaaacatc atccctaact caactggtgc tgcgaaagca 600

atcggtttag ttattcctga aatcgatggt aaattagacg gtggtgcaca acgtgttcct 660

gtagcaacag gttcattaac tgaattaact gtagtattag aaaaagaagt ctctgttgaa 720

gaagttaaca aagtcatgaa agaggcaact aacgaatcat tcggttacac tgaagacgaa 780

atcgtttctt cagacgttgt tggtatgact ttcggtgctt tattcgacgc aactcaaaca 840

cgtgtaatga ctgttggcga ccgtcaatta gttaaagttg 880

<210> 36
<211> 880
<212> DNA
<213> Staphylococcus epidermidis

<400> 36
gcatttagaa gaattcaaga cgtagaaggt ttagaagtgg ttgcagtaaa cgacttaact 60

gatgacgata tgttagcaca cttattaaaa tatgacacta tgcaaggtcg cttcactggt 120

gaagtagaag ttatcgacgg tggattccgt gttaacggta aagaagttaa atcattcgat 180

gaaccagatg caagcaaatt accttggaaa gacttagata tcgacgtagt attagaatgt 240

actggtttct atactgacaa agataaagca caagctcatg ttgacgcagg tgctaaaaaa 300

gtattaatct cagctccagc aactggtgac ttaaaaacaa tcgtttacaa cactaaccac 360

gatgaattag atggttctga aacagtagta tcaggtgcat catgtactac taactcatta 420

gctcctgtag ctaaagtgtt aagtgatgag ttcggtttag ttgaaggttt aatgacaact 480

atccacgctt acactggtga ccaaaatact caagatgctc cacacagaag aggcgacaaa 540

cgtcgtgctc gtgctgcagc agaaaacatc atccctaact caactggtgc tgctaaagca 600

atcggtaaag taattcctga aatcgacgga aaattagacg gtggtgcaca acgtgttcca 660

gttgcaactg gttcattaac tgaattaaca gttgtattag aaaaagacgt aacagttgaa 720

caagttaacg aagcaatgaa acaagcttct gacgaatcat tcggttacac tgaagacgaa 780

atcgtatctt ctgatgtagt tggtatgact tacggttcat tattcgatgc aactcaaact 840

cgtgtaatgg ctgttggtgg tcgtcaatta gttaaagtag 880

<210> 37
<211> 880
<212> DNA
<213> Staphylococcus equorum

<400> 37
gcatttagaa gaattcaaga cgtagaaggt attgacgtag tagcagttaa cgatttaaca 60

gatgacgaaa tgttagctca tttattaaaa tatgacacta cacaaggtcg cttcacagga 120

gaagttgaag tagaaaaaga cggattccgt gtaaatggac aagaagttaa atcattctca 180

gaacctgaac caagtaaatt accttggaaa gatttagaca tcgatgttgt tttagaatgt 240

actggtttct tcgctgataa agaaaaagca gaagctcaca ttgacgctgg cgctaaaaaa 300

gtattaatct ctgcaccagc aacaggcgac ttaaaaacaa tcgtttataa cactaaccac 360

agtgaattag atggttcaga aacagttgtt tcaggtgctt catgtactac taactcatta 420

gctccagtag ctaaagtttt aaatgacgac ttcggcttag ttgaaggttt catgactact 480

attcacgcat atactggtga ccaaagtact caagatgctc cacacagaaa aggcgacaaa 540

cgccgtgcac gtgcagctgc tgaaaacatc atccctaact caacaggtgc tgctaaagca 600

attggtttag taatccctga aatcgatggt aaattagacg gtggcgctca acgtgttcca 660

gttgctactg gttcattaac tgaacttaca gttgtattag aaaaagacgt aagcgttgaa 720

gacgttaacg cagcaatgaa aaatgcttca gacgaatcat ttggttacac tgaagacgaa 780

atcgtttctt ctgacgtaat cggtatgact tacggttcat tattcgatgc aacgcaaact 840

cgtgttatga cagttggaga tcaccaatta gttaaaatag 880

<210> 38
<211> 880

<212>  DNA
<213>  Staphylococcus gallinarum

<400>  38
gcattcagaa gaattcaaaa cgttgaagga atcgacgtag tagcagtaaa tgacttaaca      60

gatgacgaaa tgttagctca cttattaaaa tatgatacta tgcaaggtcg cttcactgga     120

gaagttgaag ttgaaaaaga cggtttccgt gttaacggtc aagaagttaa atcattctct     180

gagccagacc caagtaaatt accatggggt gacttagaca tcgatgtagt attagaatgt     240

actggtttct tcgctgacaa aactaaagca gaagctcaca tcaatgcagg tgctaaaaaa     300

gtattaatct cagctccagc aactggtgac ttgaaaacaa tcgttttcaa cactaaccat     360

aacgaattag atggtacaga aacagttgtt tcaggtgctt catgtactac taactcatta     420

gctccagtag ctaaagtatt aaatgatgac tttggtttag ttgaaggtct tatgactaca     480

attcacgctt acactggtga ccaaaataca caagatgctc cacatgctaa aggtgacaaa     540

cgccgtgctc gtgcagctgc tgaaaatatc atccctaact caactggtgc tgctaaagca     600

atcggtaaag ttatccctga aattgacggc aaattagacg gtggtgcgca acgtgtacca     660

gttgctactg gttctttaac tgaattaaca gttgtattag aaaaagacgt aagcgttgaa     720

gacgttaaca atgcaatgaa aaacgcttca aacgaatcat tcggttacac tgaagacgaa     780

atcgtatctt ctgacgtagt tggtatcact tacggtccat tattcgatgc aacacaaact     840

cgtgtaatga ctgttggcga tcgtcaatta gttaaagttg                           880

<210>  39
<211>  880
<212>  DNA
<213>  Staphylococcus haemolyticus

<400>  39
gcatttagaa gaattcaaga cgtaggaggt attgaagtag ttgcagtaaa cgacttaaca      60

gacgacgaaa tgttagctca tttattaaaa tatgacacta tgcaaggtcg tttcacagga     120

gaagttgaag ttgttgatgg tggtttccgc gtaaatggta aagaagttaa atcatacgaa     180

gaaccagatg caagcaaatt accttggggc gatttagata tcgacgtagt attagaatgt     240

actggtttct atacagataa agaaaaagca gaagcacaca tcaatgcagg tgctaaaaaa     300

gtattaatct ctgcaccagc tcaaggtgat gtaaaaacaa tcgtattcaa cactaaccac     360

aatgacttag atggttcaga aacagttgtt tcaggtgcat catgtactac taactcatta     420

gcaccagttg ctaaagtatt aagtgatgaa tttggtttag ttgaaggttt aatgacaact     480

attcacgcat acactggtga ccaaatgact caagacggtc cacataaaaa aggtgacaaa     540

cgtcgtgcac gtgcagcagc tcaaaacatc gtacctaact caacaggtgc tgcaaaagca     600

atcggtaaag ttattcctga aatcgatggt aaattagacg gtggtgctca acgtgtacca     660

gttgctacag gttcattaac tgaagtgaca gttgtattag aaaaagacgt tactgttgaa 720

gacgttaaca aagcaatgaa aaacgcttca aacgaatcat ttggttacac tgaagacgaa 780

atcgtttctt ctgacgtagt tggcatgact tacggttcat tattcgatgc tactcaaact 840

cgtgtaatgt ctgttggtga ccgtcaatta gttaaagttg 880

<210> 40
<211> 880
<212> DNA
<213> Staphylococcus hominis

<400> 40
gcattcagaa gaattcaaga cgtagaaggt attgaagtag ttgcagtaaa cgacttaaca 60

gacgacgaaa tgttagctca tttattaaaa tatgacacta tgcaaggtcg ctttaacgga 120

gacgtagaag tagttgaagg tggtttccgt gtaaatggta aagaagttaa atcttttgaa 180

gaaccagatg caagtaaatt accatggggc gatttagata tcgacgtagt attagaatgt 240

actggtttct atacagaaaa agaaaaagct gaagcacaca ttaatgcagg agctaaaaaa 300

gtattaattt ctgctccagc caaaggtgat gttaaaacta tcgtatttaa cacaaaccac 360

aaagacttag atggatctga aacagtagta tcaggtgctt catgtactac aaactcatta 420

gcaccagttg ctaaagtttt aaatgacgaa tttggtattg ttgaaggttt aatgacaact 480

atccatgctt acactggtga ccaaatgact caagatggtc cacacagaaa aggtgacaaa 540

cgtcgtgcac gtgcagcagc acaaaacatc gtacctaact caacaggtgc agctaaagct 600

atcggtaaag taattcctga aatcgatggt aaattagatg gtggagcaca acgtgtacca 660

gtagctactg gttcattaac tgaagtaaca gttgtattag aaaaagaagt aacagttgaa 720

gatgttaaca aagcaatgaa aaatgctgct gacgaatcat tcggttacac tgaagatgaa 780

atcgtatcat cagatgttgc tggtatgaac tttggttcat tattcgatgc aactcaaact 840

cgtgtcatct cagttggcga caaacaatta gttaaagtag 880

<210> 41
<211> 880
<212> DNA
<213> Staphylococcus hyricus

<400> 41
gcatttagaa gaattcaaga cgtagaaaac attgaggtag tagctgtcaa tgatttaact 60

gacgacgaca tgcttgcaca tttattaaaa tatgacacga tgcaaggacg ttttactgaa 120

gaagtagatg taattgatgg tggtttccgc gtaaatggta aagaagtgaa atcattctct 180

gaaccagaac catctaaatt accttggaaa gacttagaag tagatgttgt tttagaatgt 240

actggtttct tcacatctaa agaaaaagct gaagcacaca ttgaagcagg cgctaaaaaa 300

gtcttaattt cagcaccagg tactggtgat cttaaaacaa ttgtatataa cgttaaccat 360

gaagaattag acggttcaga aacagttgtt tcaggtgcgt cttgtactac aaactcatta 420

gctccggtag cgaaaacatt acacgatgaa tttggtattg ttgaaggttt aatgactaca 480

attcacgctt atacaggtga ccaaaatacg caagactcac ctcacagaaa aggtgacaaa 540

cgtcgtgcac gtgcagctgc tgaaaacatc atccctaact caacaggtgc tgcaaaagca 600

atcggtttag ttattccaga aattgctggt aaattagatg gtggcgcgca acgtgtacca 660

gttgctacag gttcattaac agaattaact gttgttttag aaaaagaagt atctgttgaa 720

gaagttaaca atgcaatgaa aaatgcaact aatgaatcat tcggttacac tgaagatgaa 780

atcgtctctt ctgacgttgt aggtatgacg tttggtgcat tattcgacgc aactcaaaca 840

cgcgttatga ctgttggcga tcgtcaatta gttaaagttg 880

<210> 42
<211> 883
<212> DNA
<213> Staphylococcus intermedius

<400> 42
gcattccgtc gtattcaaaa tgtggaagga attgaagttg ttgcaatcaa tgacttaaca 60

gacgctaaaa tgttagctca tctgttaaaa tatgatacaa ctcaaggccg ttttgacggt 120

gaagtagaag tacatgatgg tttcttcaaa gtaaacggta aagaagttaa agtattagct 180

aaccgtaacc cagaagaact tccatggggt gaactaggag tagacatcgt tcttgaatgt 240

actggtttct tcacagcaca agacaaagct gaattacaca ttaaagctgg cgctaaaaaa 300

gttgttatct ccgctccagc aactggcgac atgaaaacaa tcgtttacaa tgtaaaccat 360

gaaacattag acggaactga aacagttatt tctggtgcaa gctgtactac taactgttta 420

gctccaatgg ctaaagtttt agaagacaaa tttggtgttg ttgaaggcct aatgactaca 480

attcacgcat acactggtga ccaaaataca ttagacgctc cacatccaaa aggtgacttc 540

cgtcgtgctc gtgctgctgc agaaaatatc atccctaata caactggtgc tgcaaaagct 600

atcggtgaag tattaccaag ccttaaaggt aaattagacg gagcagctca acgtgttcca 660

gttccaactg gttcccttac tgaattagta acagttctta acaaaaaagt tactgttgat 720

gaagtaaatg cagctatgga agcagcttct gatccagaaa cattcggtta cactaatgac 780

gcaatcgttt cttctgatat caaaggtatg actttcggtt ctttatttga cgaaactcaa 840

acaaaagttc ttacagttgg cgatcaacaa ttagttaaaa ctg 883

<210> 43
<211> 881
<212> DNA
<213> Staphylococcus kloosii

<400> 43

gcatttagaa gaattcaaaa cgttgacgga atcgatgtag tagcagttaa cgacttaaca 60

gatgacgaaa tgttagcaca cttattaaaa tatgacacaa tgcaaggtcg tttcactgga 120

gaagttgaag ttgaagaaaa cggcttccgc gtaaatggtc aagaagttaa atcattctct 180

gaaccagatc caagtaaatt accatggggc gacttagata tcgatgttgt cttagaatgt 240

actggtttat ttgctgataa agataaagct tcagctcata tcgatgcagg cgctaaaaaa 300

gttttaattt cagctccagc tacaggcgac ttaaaaacaa tcgtttacaa cactaaccac 360

aacgaattag acggttcaga atcagtagta tcaggtgctt catgtactac taactcatta 420

gctccagtag ctaaagtttt aaatgatgaa tttagtttag ttgaaggttt aatgacaact 480

atccacgctt acactggtga ccaaagcaca caagatgctc ctcacagaaa aggtgacaaa 540

cgtcgtgctc gtgcagcagc agaaaacatc atccctaact caacaggtgc tgcaaaagca 600

atcggtttag ttattcctga aatcgacgga aaattagacg gtggcgcaca acgtgttcca 660

gttgcaacag gttcattaac tgaattaaca gttgtattag aaaaagacgt aagtgttgaa 720

gatgtaaaca acgcaatgaa aaatgcttca aacgaatcat ttggttacac tgaagacgaa 780

atcgtttctt ctgacgtaat cggtatgact tacggttcat tattcgacgc tacacaaact 840

cgtgtaatga ctgttggtga ctcgtcaatt agttaaagtt g 881

<210> 44
<211> 880
<212> DNA
<213> Staphylococcus lentus

<400> 44
gcatttagaa gattacaaga agtagaaaat atcgaagtag tagcaatcaa cgatttagca 60

gatgacgcta tgttagctca tttattaaaa tacgattcta cacaaggtcg tttcaaagat 120

gaagtagaag taattgaagg cggattccgt gtaaacggta aagaaatcaa aactttcgaa 180

aatcctaacc ctaaagaatt accctgggga gacttagaca tcgatgtagt attagaatgt 240

actggtttct tcgctgataa agaaaaagct catgctcaca tcgccgcagg tgctaaaaaa 300

gtattaattt cagctccagc ttcaggcgac ttgaaaacaa tcgtatacaa tgttaaccat 360

gatgaattag acggttcaga agaaatcgta tctggtgcat cttgtactac taactgttta 420

gctccaatgg ctaaagtatt aaatgatgaa ttcggtatcg ttgaaggatt aatgatgaca 480

attcatgctt atactggtga ccaaaataca ctagatgctc cacatgctaa aggtggcttc 540

cgtcgtgctc gtgcagcagc tgaaaacatc gtacctaact caactggtgc agctaaagca 600

attggcttag ttatcccaga attaaaaggt aaattagatg gatcagctca acgtgttcca 660

gtagcaactg gttcagtaac tgaattaaca gcagtattag ataaagaagt atctatagaa 720

gaaatcaacg aagcaatgaa aaatgctaca aatgattcat tcggatacac tgaagacgaa 780

atcgtttctt ctgatgttat tggcatcact tacggttcat tattcgacgc aactcaaact 840

cgtgtaatga cagttggaga ccgtcaatta gttaaaactg 880

<210> 45
<211> 883
<212> DNA
<213> Staphylococcus lugdunesis

<400> 45
gcatttagaa gaattcaaga cgtagaaggc atcgaggtag tagcagtaaa cgacttaaca 60

gatgatgata tgttagcgca tttattaaaa tatgacacta tgcaaggtcg cttcactagc 120

gaagttgaag ttgttgatgg tggtttccgt gtaaatggta aagaagttaa atcatttgaa 180

gaaccagacg caagcaaatt accatggggt gacctaggtg ttgacgtggt attagaatgt 240

actggattct atacagataa agaaaaagct gaagcacaca ttcatgcagg tgctaaaaaa 300

gtattaattt ctgcgccagc taaaggtgac gttaaaacta tcgtttacaa cactaaccat 360

agtgacttag acggttcaga aacagttgta tcaggtgctt catgtacaac taactcttta 420

gcaccagtag ctaaagtaat cagcgatgaa tttggtttag tagaaggttt aatgacaact 480

attcatgcat acactggtga ccaaatgact caagatggtc cacacagaaa aggtgacaaa 540

cgtcgtgctc gtgcagctgc acaaaacatc gtacctaact caactggtgc tgctaaagca 600

atcggtaaag ttattcctga aatcgatggt aaattagacg gtggtgcgca acgtgtacct 660

gtagctacag gttcattaac tgaattaact gtagtattag aaaaacaaga tgtaacagta 720

gaacaagtaa acgaagcgat gaaaaaagct tctaacgaat cattcggtta caatgaagat 780

gaaattgttt cttctgacgt agttggtatg acttacggtt cattatttga tgcaacacaa 840

actcgtgtaa tgtcagttgg tggccgtcaa ttagttaaag ttg 883

<210> 46
<211> 880
<212> DNA
<213> Staphylococcus piscifermentans

<400> 46
gcattcagaa gaattcaaga tgttgaaggt atcgatgtag tagcagtaaa cgacttaaca 60

gatgatgaaa tgttagcgca tttattaaaa tacgatacaa tgcaaggacg tttcacagaa 120

gaagttgaag ttgtagatgg cggattccgt gtgaatggta aagaagttaa atcattcgaa 180

gaaccagatg caagcaaatt accttggaaa gatttagaca ttgatgttgt attagaatgt 240

actggtttct atacaagtga tgaaaaagct aaagcacata tcgacgcagg tgctaaaaaa 300

gtattaattt ctgctccagc aactggcgat gttaaaacaa tcgtttataa cgtaaaccaa 360

gatactttag acagctctga tgttatcgtt tcaggtgctt cttgtactac aaactcactt 420

gctccagtag caaaagtttt aaacgacagc tttggtttag ttgaaggttt catgactact 480

attcatgctt acactggtga ccaaaatact caagatggtc cacacagaaa aggtgacaaa 540

cgtcgtgcac gtgcagcagc tcaaaacatc gtaccaaact caactggtgc tgctaaagca 600

atcggtaaag taatccctga aattgacggt aaattagacg gtggtgctca acgtgttcct 660

gttgcaactg gttcattaac agaattaaca gttgtattag acaaagaagt ttcagttgac 720

gaagtaaacg aagcaatgaa acaagcttct aacgaatcat tcggttacaa tgaagacgaa 780

atcgtatctt ctgacgtggt tggtatgaca ttcggttcat tattcgatgc tacacaaact 840

cgtgtgatga ctgtatcagg tcgtcaatta gttaaagttg 880

<210> 47
<211> 880
<212> DNA
<213> Staphylococcus saprophyticus

<220>
<221> misc_feature
<222> (701)..(707)
<223> n = a or g or c or t/u, unknown or other

<400> 47
gcattcagaa gaattcaaaa cgttgacgga atcgacgtag tagcagtaaa cgatttaaca 60

gatgacgaaa tgttagctca tttattaaaa tatgatacta tgcaaggacg cttcacagga 120

gaagttgaag tagaaaaaga cggtttccgc gtaaacggac aagaagtaaa atcattctct 180

gagcctgaac caagtaaatt accttggaaa gacttagaca tcgatgttgt attagaatgt 240

actggtttct tcgctgataa agaaaaagca gaagcacaca tcaatgcagg tgctaaaaaa 300

gtattaatct ctgctccagc tacaggcgat ttaaaaacaa tcgtttataa tacaaaccac 360

caagaattag acggttcaga aactgttgtt tcaggtgctt catgtactac taactcatta 420

gctcctgttg ctaaagtttt aaatgatgac ttcggtttag tagaaggttt catgactact 480

atccacgcat acactggtga ccaaagcaca ctagatgcac cacacagaaa aggcgacaaa 540

cgtcgtgcgc gtgcagctgc tgaaaacatc atccctaact caactggtgc tgctaaagca 600

attggcttag taattcctga aattgatggt aaattagatg gaggagcgca acgtgttcct 660

gttgcaactg gttcattaac tgaattaaca gttgttttag nnnnnnntgt aagcattgaa 720

gatgtaaatg ctgcaatgaa aaatgcttca aacgaatcat tcggttacac agaagacgaa 780

atcgtatctt cagacgtaat cggtatgact tatggttcat tatttgatgc aacacaaact 840

cgtgtaatga ctgttggcga ccgtcaatta gttaaagtag 880

<210> 48
<211> 880
<212> DNA
<213> Staphylococcus schleiferi

<400> 48
gcatttagaa gaattcaaga tgtagaaaac attgaggtcg tagctgtaaa cgatttaaca 60

gatgacgata tgcttgcaca tttattgaaa tatgacacaa tgcaaggacg ttttactgaa 120

gaagtggaag taattgatgg tggtttccgt gtgaatggta aagaagcgaa atcattctct 180

gaaccagaac ctgctaaatt accttggggt gaccttggtg tggacgtagt attagaatgt 240

actggtttct tcacagataa agaaaaagct gaagcacaca ttcaagcagg cgctaaaaaa 300

gtattaatct cagcaccggc taaaggtgat cttaaaacaa tcgtatataa tgttaaccac 360

gacgatttag atggttctga aacagttgtt tcaggtgcat catgtactac aaactcatta 420

gcacctgttg caaaaacttt acacgacgaa ttcggtattg ttgaaggttt aatgactaca 480

attcacgcat atactggtga ccaaaataca caagatgcac ctcacagaaa aggtgacaaa 540

cgtcgtgcgc gtgctgctgc tgaaaacatt atccctaact ctacaggggc agcaaaagca 600

atcggtttag ttattccaga aattgccggt aaattagacg gtggtgcaca acgtgttcca 660

gttgcaactg gttcattaac agaattaaca gttgttttag ataaagaagt gactgttgaa 720

gaagtaaaca aagtattgaa agcagcaact aacgaaccat tcggttacac tgaagacgaa 780

attgtttctt cagacgttgt aggtatgact tacggtgcat tattcgatgc aactcaaact 840

cgtgtaatga ctgttggcga ccgtcaatta gttaaagttg 880

<210> 49
<211> 880
<212> DNA
<213> Staphylococcus sciuri

<400> 49
gcatttagaa gattacaaga agttgaaaat atcgaagtag tagcaatcaa cgatttaaca 60

gatgacgcaa tgttagctca tttattaaaa tatgattcaa cacaaggtcg tttcaaagac 120

gaagtagaag ttatcgaagg cggattccgc gtaaacggta gagaaatcaa aactttcgaa 180

aatcctaatc ctaaagaatt accatggggc gatttagata tcgatgtagt attagaatgt 240

actggtttct tcgctgacaa agacaaagct tcagctcaca tcgaagcagg tgctaaaaaa 300

gtattaattt cagctccagc atcaggtgac ttaaaaacta tcgtttataa cgttaaccat 360

gacgaattag acggatctga agaaatcgtt tcaggtgcat cttgtacaac taactgttta 420

gctccaatgg ctaaagtatt aaatgatgaa ttcggtatcg ttgaaggttt aatgatgaca 480

attcacgctt acactggtga ccaaaatact ttagatgctc cacatgctaa aggtgacttc 540

cgtcgtgctc gtgcagctgc tcaaaacatc gtgcctaact caactggtgc tgctaaagca 600

atcggtttag taattccaga attaaaaggt aaattagatg gatcagctca acgtgttcca 660

gtagcaactg gttcagtaac agaattaaca gctgtattgg acaaagaagt ttcagttgaa 720

gaaatcaatg cagcaatgaa aaatgctaca aatgattcat tcggttacac tgaagacgaa 780

atcgtatctt ctgatatcat tggtatcact tacggttcat tatttgatgc aactcaaact 840

cgtgttatga cagttggaga tcgccaatta gttaaaactg 880

<210> 50
<211> 881
<212> DNA
<213> Staphylococcus simulans

<400> 50
gcatttcaga agaattcaag atgttgaagg tatcgatgta gtagcagtaa acgacttaac 60

agatgatgaa atgttagcac acttattaaa atacgataca atgcaaggac gtttcactga 120

agaagttgaa gttgtagatg gcggattccg cgtgaatggt aaagaagtta aatcattcga 180

agaaccagat gcaagcaaat taccttggaa agatttagac attgatgtcg tattagaatg 240

tactggtttc tacactagcg acgaaaaagc acaagctcac attgacgcag gtgctaaaaa 300

agtattaatc tctgcaccag caactggtga cgttaaaaca atcgtttata acgtaaacca 360

agatacttta gacagctctg acgtaatcgt ttcaggtgct tcttgtacta caaactcact 420

tgctccagta gcaaaagtat taaatgacag cttcggttta gtagaaggtt tcatgactac 480

tatccacgct tacactggtg accaaaatac tcaagacggt ccacacagaa aaggcgacaa 540

acgtcgtgca cgtgcagcag ctgaaaacat cgttcctaac tcaactggtg ctgctaaagc 600

aatcggtaaa gtaattcctg aaatcgacgg aaaattagac ggtggcgctc aacgtgttcc 660

tgtagcaact ggttcattaa ctgaattaac agttgtatta gacaaagacg taacaatcga 720

agaagtaaac gaagctatgg aagcagcttc taacgaatca ttcggttaca acgaagacga 780

aatcgtatct tcagacgtag ttggtatgac attcggttca ttattcgatg caactcaaac 840

tcgtgttatg actgtatctg gtcgtcaatt agttaaagtt g 881

<210> 51
<211> 880
<212> DNA
<213> Staphylococcus xylosus

<400> 51
gcatttagaa gaattcaaaa cgttgacgga attgacgtag tagcagtaaa tgacttaaca 60

gatgacgaaa tgttagcaca tttattaaaa tatgacacta tgcaaggacg cttcacagga 120

gaagttgaag tagaaaatga cggtttccgt gtaaacggac aagaagtaaa atcattctct 180

gagccagacc caagcaaatt accttggaaa gatttagaca tcgatgttgt attagaatgt 240

actggtttct acgctgataa agaaaaagca gaagctcaca ttaatgcagg tgctaaaaaa 300

gtattaattt cagctccagc tactggtgat ttaaaaacaa tcgtttataa cacaaaccac 360

caagagttag atggtaaaga aacagttgtt tcaggtgctt catgtacaac taactcatta 420

gctccagttg ctaaagtatt aaatgatgac tttggtttag tagaaggttt catgactaca 480

attcatgctt acactggtga ccaaaataca caagatgcgc cacacgctaa aggcgacaaa 540

cgtcgtgctc gtgcagctgc tgaaaacatt atccctaact caactggtgc tgctaaagca 600

attggcctag ttatccctga aattgatggt aaattagacg gtggagcgca acgtgttcct 660

gtagctactg gttcgttaac agaattaaca gttgtattag acaaaaatgt aagtgttgaa 720

gacgttaatg ctgcaatgaa gaatgcttca aacgaatcat tcggttacac tgaagacgaa 780

atcgtttctt ctgatgtagt tggtatgact tacggttcat tattcgatgc aacacaaaca 840

cgtgttatga cagttggcga tcgccaatta gttaaagtag 880

<210> 52
<211> 696
<212> DNA
<213> Staphylococcus caseolyticus

<220>
<221> misc_feature
<222> (657)..(657)
<223> n = a or g or c or t/u, unknown or other

<400> 52
ttagaacagc atgcacgggt aaaagcattg gatgcacaat atccagaaat tgatatttat 60

tcaggtgttg agatggatat attagcagat ggcgaaatgg attatagcaa tgacgtgcta 120

gcacagcttg actattgtat tggtgctatt catcagtcgt tgaatcaaag tgaagatgag 180

attatgaaac gcctgatcaa tgcctgtaat aatccataca ttagacatat tgctcatcca 240

acgggaaggt tgattggtcg ccgtaatggt tatcatgtaa acatgccgaa actcatcgaa 300

actgcacaga agacaaatac catccttgaa atcaatgcac atccgatgcg ccttgattta 360

tcgagcgacg tattaaagca atatccagat attaaacttg tgatcaacac agacgcgcgt 420

gcaatcgatc agcttgattt aatgaaatat ggtgtgggta cagcaataaa aggccatgtg 480

aaaaaagaac aggtaataaa cactttaccg cgtaaggatt ttaaatcttg gatacagaat 540

gggaagtaat tatatgaata aaaaagcatt aaatatatta gaatataaca aaattattga 600

gcgtgttgac gcatttactc aaaatgaact ttcaagcaaa aagtgaggat gacacancct 660

atcagcgaca aagctgaaat agatagcatg cttgca 696

<210> 53
<211> 26
<212> DNA
<213> Artificial Sequence

<220>
<223> primer

<400> 53
atggttttgg tagaattggt cgttta 26

<210> 54
<211> 25
<212> DNA
<213> Artificial Sequence

<220>
<223> primer

<400> 54
gacatttcgt tatcatacca agctg 25

<210> 55
<211> 26
<212> DNA
<213> Artificial Sequence

<220>
<223> primer

<400> 55
atggttttgg tagaattggt cgttta 26

<210> 56
<211> 25
<212> DNA
<213> Artificial Sequence

<220>
<223> primer

<400> 56
gacatttcgt tatcatacca agctg 25